(19) Europäisches Patentamt / European Patent Office / Office européen des brevets

(11) EP 2 851 675 B1

(12) EUROPEAN PATENT SPECIFICATION

(45) Date of publication and mention of the grant of the patent:
**06.02.2019 Bulletin 2019/06**

(21) Application number: **13790271.4**

(22) Date of filing: **13.05.2013**

(51) Int Cl.:
*G01N 21/27* (2006.01)    *F16H 57/04* (2010.01)
*G01N 33/30* (2006.01)

(86) International application number:
**PCT/JP2013/063307**

(87) International publication number:
**WO 2013/172306 (21.11.2013 Gazette 2013/47)**

(54) DECELERATOR DAMAGE STATE NOTIFICATION DEVICE, MECHANICAL SYSTEM WITH DECELERATOR DAMAGE STATE NOTIFICATION FUNCTION, AND MEDIUM ON WHICH DECELERATOR DAMAGE STATE NOTIFICATION PROGRAM IS RECORDED

VORRICHTUNG ZUR MELDUNG DES SCHADENSSTATUS EINES ENTSCHLEUNIGERS, MECHANISCHES SYSTEM MIT FUNKTION ZUR MELDUNG DES SCHADENSSTATUS EINES ENTSCHLEUNIGERS UND MEDIUM MIT DARAUF AUFGEZEICHNETEM PROGRAMM ZUR MELDUNG DES SCHADENSSTATUS EINES ENTSCHLEUNIGERS

DISPOSITIF DE NOTIFICATION DE L'ÉTAT DE DÉGRADATION D'UN DÉCÉLÉRATEUR, SYSTÈME MÉCANIQUE ÉQUIPÉ D'UNE FONCTION DE NOTIFICATION DE L'ÉTAT DE DÉGRADATION D'UN DÉCÉLÉRATEUR ET SUPPORT D'ENREGISTREMENT D'UN PROGRAMME DE NOTIFICATION DE L'ÉTAT DE DÉGRADATION D'UN DÉCÉLÉRATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2012 JP 2012113127**

(43) Date of publication of application:
**25.03.2015 Bulletin 2015/13**

(73) Proprietor: **Nabtesco Corporation Tokyo 102-0093 (JP)**

(72) Inventors:
• **NAKAMURA, Koji**
  **Tsu-shi, Mie 514-8533 (JP)**
• **SHIMADA, Hideshi**
  **Tsu-shi, Mie 514-8533 (JP)**

(74) Representative: **Grünecker Patent- und Rechtsanwälte**
  **PartG mbB**
  **Leopoldstraße 4**
  **80802 München (DE)**

(56) References cited:
| EP-A1- 2 261 534 | EP-A1- 2 447 704 |
| WO-A1-2012/074112 | JP-A- H 107 323 |
| JP-A- 2007 192 769 | JP-A- 2007 212 161 |
| JP-U- H0 635 984 | US-A1- 2008 024 761 |
| US-A1- 2009 216 464 | |

## Description

Technical Field

The present invention relates to a reducer damage state notification device for sending notification relating to a damage state of a reducer of a machine which includes the reducer and a lubricant deterioration sensor for detecting the deterioration of lubricant of the reducer.

Background Art

The closest prior art document EP2447704A1 (D1) discloses an oil state monitoring method and an oil state monitoring device for monitoring the state of degradation of oil used in machinery or equipment. Additionally, D1 discloses that after detecting the color components of first transmitted light and the first reflected light as well as of the second transmitted light and the second reflected light, the maximum color difference will be calculated. Further, D1 describes that when calculation has been done, then the control device judges the oil degradation states and displays in the next step color data and judgment result that the oil is at the end of its life and should be replaces with new oil or that the oil is continuously usable, and so on, on a monitor (S122).

In the related art, as a lubricant deterioration sensor for detecting the deterioration of lubricant of a machine, an oil deterioration degree sensor is known. In this sensor, an oil entering gap portion for making lubricant enter therein is formed on an optical path from an infrared light emitting diode (LED) to a photo diode, an amount of light absorption by the lubricant within the oil entering gap portion with respect to light emitted from the infrared LED is measured according to a light reception amount of the photo diode, and thus, a deterioration degree of the lubricant related to the measured light absorption amount is determined (for example, refer to PTLs 1 and 2).

However, in the oil deterioration degree sensors disclosed in PTLs 1 and 2, there is a problem that although a density of insoluble content within the lubricant can be measured as the deterioration degree of the lubricant, kinds of contaminants within the lubricant cannot be specified.

As a technique of specifying the kinds of contaminants within the lubricant, a technique is known, in which light is radiated to a membrane filter by an LED after the lubricant is filtered, reflection light from the contaminant on the membrane filter is converted into RGB digital values by a light reception element, and the kinds of contaminants within the lubricant are specified based on the converted RGB digital values (for example, refer to NPLs 1 and 2).

Citation List

Patent Literature

[PTL 1] JP-A-7-146233
[PTL 2] JP-A-10-104160

Non Patent Literature

[NPL 1] Tomohiko Yamaguchi and four others, "METHOD OF DISCRIMINATING HUE OF CONTAMINANT WITHIN LUBRICANT", Fukui University, Faculty of Engineering, Research Report, March 2003, Volume 51, No. 1, pp. 81 - 88
[NPL 2] Tomomi Honda, "DETERIORATION DIAGNOSIS OF LUBRICANT · INSPECTION TECHNOLOGY", Journal of Japan Society for Precision Engineering, 2009, Volume 75, No. 3, pp. 359 - 362

Summary of Invention

Technical Problem

In the techniques disclosed in NPLs 1 and 2, it is necessary to drain the lubricant from the machine and filter the lubricant through the membrane filter, and thus, there is a problem that immediacy is lacking.

The inventors have invented a lubricant deterioration sensor which is included in a machine having a reducer and can immediately specify kinds and an amount of contaminant within lubricant of a reducer. The lubricant deterioration sensor (hereinafter, referred to as a "new sensor") includes a light emitting element which emits light, a color light reception element which detects color of received light, and a gap forming member in which an oil gap, which causes the lubricant of the reducer to enter therein and is disposed on an optical path from the light emitting element to the color light reception element, is formed, and by which the light is transmitted.

Moreover, the inventors have invented a reducer damage state notification device which sends the notification relating to the state of damage of the reducer corresponding to a threshold value when a color difference between a color detected by the color light reception element of the new sensor and a predetermined color reaches a predetermined threshold value.

However, the inventors of the present application have found that a relationship of a color difference between the color detected by the color light reception element of the new sensor and a predetermined color, and the damage state of the reducer becomes different according to the use condition of the reducer such as a load applied to the reducer. When the relationship of the color difference between the color detected by the color light reception element of the new sensor and a predetermined color, and the damage state of the reducer is not constant, there is a problem that the reducer damage state notification device cannot accurately send notification relating to the damage state of the reducer.

An object of the present invention is to provide a reducer damage state notification device capable of accurately sending notification relating to the damage state of the reducer even when the use conditions of the reducer are different from each other.

Solution to Problem

According to an advantage of the present invention, there is provided a reducer damage state notification device for sending notification relating to a damage state of a reducer of a machine which includes the reducer and a lubricant deterioration sensor for detecting deterioration of lubricant of the reducer,
wherein the lubricant deterioration sensor includes a light emitting element which emits light, a color light reception element which detects color of received light, and a gap forming member in which an oil gap, which the lubricant enters and is disposed on an optical path from the light emitting element to the color light reception element, is formed, and by which the light is transmitted,
the reducer damage state notification devicecomprising:

a damage state notification means for sending notification relating to the damage state of the reducer;
a threshold value setting means for setting a threshold value for notification by the damage state notification means; and
a load receiving means for receiving a load which is applied to the reducer,
wherein the damage state notification means sends notification relating to the damage state of the reducer corresponding to the threshold value when a color difference between the color detected by the color light reception element and a predetermined color reaches the threshold value set by the threshold value setting means, and
the threshold value setting means sets the threshold value according to the load which is received by the load receiving means.

The reducer may include a case which includes an internal gear, a supporting body which is rotatably supported by the case, an external gear which meshes with the internal gear, and a crank shaft which is rotatably supported by the supporting body and the external gear and is configured to eccentrically rotate the external gear with respect to the case according to a rotational motion input from the outside, and the load receiving means may receive at least one of a load which is applied to the reducer in a rotation direction about a rotation axis when the case and the supporting body are relatively rotated, and a load which is applied to the reducer in a rotation direction about an axis orthogonal to the rotation axis.

The machine may include a load sensor which is configured to detect the load applied to the reducer, and the load receiving means may receive the load which is detected by the load sensor.

The threshold value setting means may set the threshold value according to an average value of predetermined periods of the load which is received by the load receiving means.

The reducer damage state notification device may include lubricant temperature receiving means for receiving a temperature of the lubricant, and the threshold value setting means may set the threshold value according to the temperature of the lubricant which is received by the lubricant temperature receiving means.

The machine may include a lubricant temperature sensor which is configured to detect the temperature of the lubricant, and the lubricant temperature receiving means may receive the temperature of the lubricant which is detected by the lubricant temperature sensor.

The threshold value setting means may set the threshold value according to an average value of the temperature of the lubricant which is received by the lubricant temperature receiving means during predetermined periods.

The threshold value may include a plurality of levels of threshold values corresponding to a possibility of the damage of the reducer.

According to another advantage of the present invention, there is provided a machine system with a reducer damage state notification function, including: the above-described reducer damage state notification device; and the machine which is configured so that the notification relating to the damage state of the reducer is sent by the reducer damage state notification device.

According to still another advantage of the present invention, there is provided a machine system with a reducer damage state notification function, comprising: the above-described reducer damage state notification device; and the machine in which the notification relating to the damage state of the reducer is sent by the reducer damage state notification device, in which the lubricant temperature sensor is incorporated in the lubricant deterioration sensor.

According to still another advantage of the present invention, there is provided a medium storing a reducer damage state notification program which causes a computer to function as the above-described reducer damage state notification device.

Advantageous Effects of Invention

In a reducer damage state notification device of the present invention, since a threshold value for sending notification relating to a damage state of a reducer is set according to a load applied to the reducer, it is possible to accurately send the notification relating to the damage state of the reducer even when the loads applied to the reducer are different from each other as a use condition of the reducer.

In the reducer damage state notification device of the present invention, since the threshold value for sending the notification relating to the damage state of the reducer is set according to at least one of a load (hereinafter, referred to as a "load torque") which is applied to the reducer in a rotation direction about a rotation axis when a case and a supporting body are relatively rotated, and a load (hereinafter, referred to as a "load moment") which is applied to the reducer in a rotation direction about an axis orthogonal to the rotation axis, it is possible to accurately send the notification relating to the damage state of the reducer even when at least one of the load torque and the load moment of the reducer is different as the use condition of the reducer.

In the reducer damage state notification device of the present invention, since the threshold value for sending the notification relating to the damage state of the reducer is set according to the load which is actually applied to the reducer, it is possible to accurately send the notification relating to the damage state of the reducer according to the load which is actually applied to the reducer.

In the reducer damage state notification device of the present invention, since the threshold value for sending the notification relating to the damage state of the reducer is set according to an average value of the loads which are actually applied to the reducer during a predetermined period, it is possible to prevent the notification relating to the damage state of the reducer from being erroneously sent even when the load applied to the reducer is temporarily and suddenly changed.

In the reducer damage state notification device of the present invention, since the threshold value for sending the notification relating to the damage state of the reducer is set according to the load applied to the reducer and a temperature of the lubricant, it is possible to accurately send the notification relating to the damage state of the reducer even when the loads applied to the reducer and the temperatures of the lubricant are different from each other as the use conditions of the reducer.

In the reducer damage state notification device of the present invention, since the threshold value for sending the notification relating to the damage state of the reducer is set according to the actual temperature of the lubricant, it is possible to accurately send the notification relating to the damage state of the reducer according to the actual temperature of the lubricant.

In the reducer damage state notification device of the present invention, since the threshold value for sending the notification relating to the damage state of the reducer is set according to an average value of the actual temperatures of the lubricant during a predetermined period, it is possible to prevent the notification relating to the damage state of the reducer from being erroneously sent even when the temperature of the lubricant is temporarily and suddenly changed.

In the reducer damage state notification device of the present invention, it is possible to send the notification relating to the damage state of the reducer in a plurality of levels as a possibility of the damage of the reducer.

In a machine system with a reducer damage state notification function of the present invention, it is possible to accurately send the notification relating to the damage state of the reducer even when the use conditions of the reducer are different from each other.

In the machine system with a reducer damage state notification function of the present invention, it is possible to accurately send the notification relating to the damage state of the reducer even when the use conditions of the reducer are different from each other. In addition, in the machine system with a reducer damage state notification function of the present invention, since a lubricant temperature sensor is incorporated in a lubricant deterioration sensor, compared to a configuration in which the lubricant temperature sensor and the lubricant deterioration sensor are separately provided, it is possible to easily configure the machine system.

In a computer of executing a reducer damage state notification program of the present invention, it is possible to accurately send the notification relating to the damage state of the reducer even when the use conditions of the reducer are different from each other.

In the reducer damage state notification device of the present invention, it is possible to accurately send the notification relating to the damage state of the reducer even when the use conditions of the reducer are different from each other.

Brief Description of Drawings

Fig. 1 is a block diagram of a machine system with a reducer damage state notification function according to an embodiment of the present invention.

Fig. 2 is a side view of an industrial robot shown in Fig. 1.

Fig. 3 is a sectional view of an articular portion of the industrial robot shown in Fig. 1.

Fig. 4 is a front view of a lubricant deterioration sensor shown in Fig. 3.

Fig. 5 is a front sectional view of the lubricant deterioration sensor shown in Fig. 4 in a state of being attached to an arm.

Fig. 6(a) is a plan view of the lubricant deterioration sensor shown in Fig. 4. Fig. 6(b) is a bottom view of the lubricant deterioration sensor shown in Fig. 4.

Fig. 7 is a view showing an optical path from a white LED to a RGB sensor shown in Fig. 5.

Fig. 8 is a block diagram of the reducer damage state notification device shown in Fig. 1.

Fig. 9 is a table showing an example of a threshold value setting table shown in Fig. 8.

Fig. 10 is a table showing an example of a table for selecting a table shown in Fig. 8.

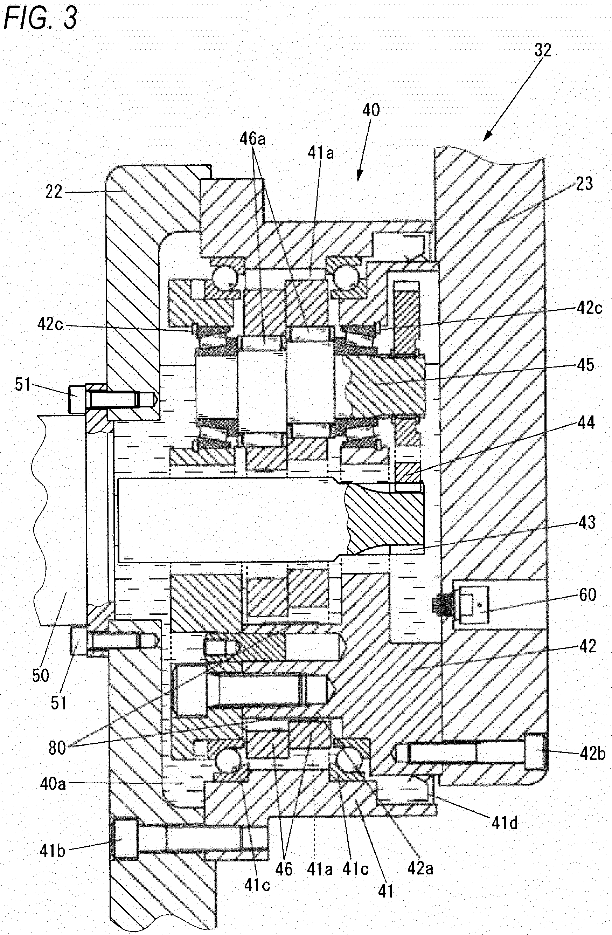

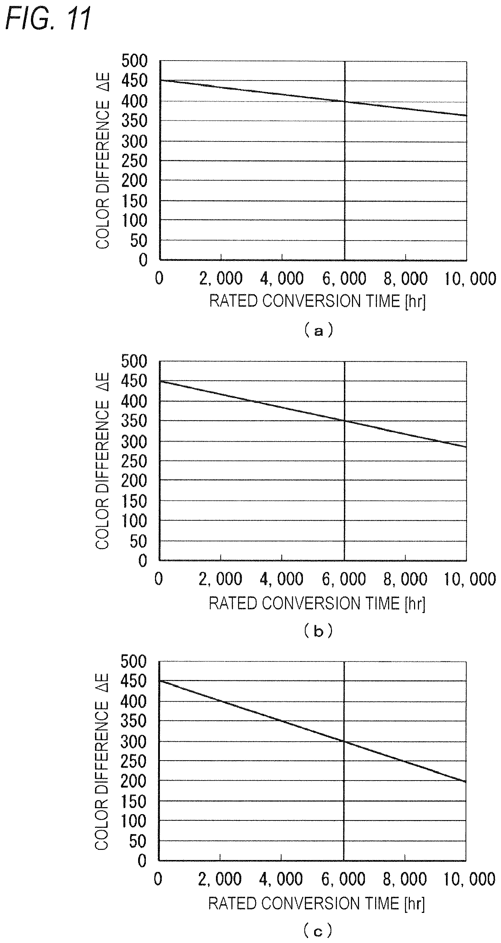

Fig. 11(a) is a graph showing an example of an experimental result of a chorological change of a color difference $\Delta E$ when a temperature of the lubricant shown in Fig. 3 is 40°C, a load moment of the reducer is 0.5 Mo, and a load torque of the reducer is 1.0 To. Fig. 11(b) is a graph showing an example of an experimentation result of a chorological change of a color difference $\Delta E$ when a temperature of the lubricant shown in Fig. 3 is 60°C, a load moment of the reducer is 0.5 Mo, and a load torque of the reducer is 1.0 To. Fig. 11(c) is a graph showing an example of an experimentation result of a chorological change of a color difference $\Delta E$ when a temperature of the lubricant shown in Fig. 3 is 60°C, a load moment of the reducer is 0.5 Mo, and a load torque of the reducer is 2.0 To.

Fig. 12 is a flowchart of an operation of the reducer damage state notification device shown in Fig. 8.

Fig. 13 is a diagram showing an example of a notification input screen displayed on a display unit shown in Fig. 8.

Fig. 14(a) is a diagram showing an example of a notification screen that warns of a high possibility of damage of the reducer on the screen displayed on the display unit shown in Fig. 8. Fig. 14(b) is a diagram showing an example of a notification screen that warns of a possibility of damage of the reducer on the screen displayed on the display unit shown in Fig. 8. Fig. 14(c) is a diagram showing an example of a notification screen that promotes service or repair of the reducer to a user on the screen displayed on the display unit shown in Fig. 8.

Description of Embodiments

Hereinafter, an embodiment of the present invention will be described with reference to drawings.

First, a configuration of a machine system with a reducer damage state notification function according to the present embodiment will be described.

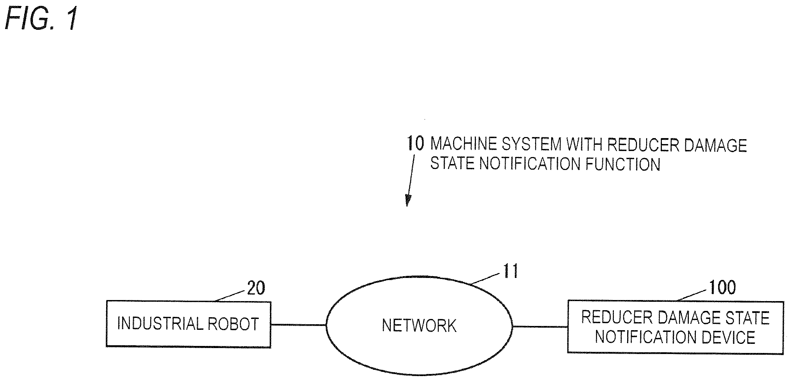

Fig. 1 is a block diagram of a machine system 10 with a reducer damage state notification function according to the present embodiment.

As shown in Fig. 1, the machine system 10 with a reducer damage state notification function includes an industrial robot 20 which is a machine of the present invention, and a reducer damage state notification device 100 for sending notification relating to a damage state of a reducer of the industrial robot 20. The industrial robot 20 and the reducer damage state notification device 100 are communicably connected to each other via a network 11 such as a local area network (LAN).

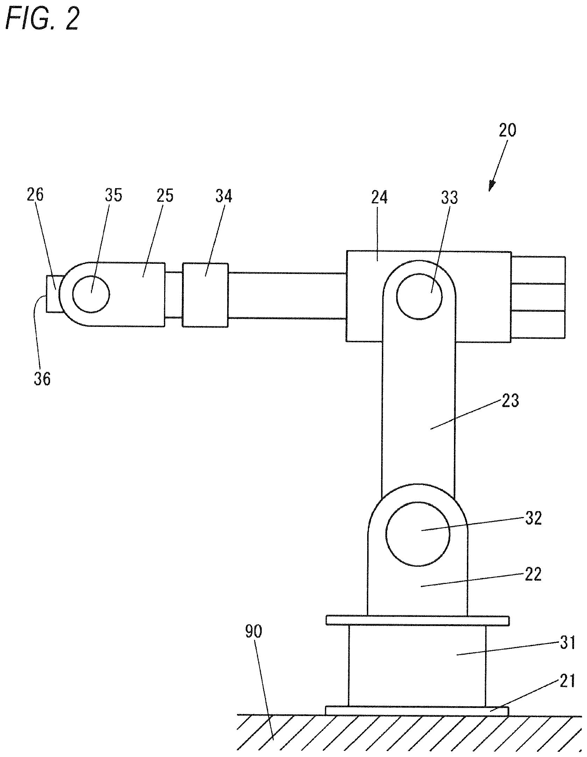

Fig. 2 is a side view of the industrial robot 20.

As shown in Fig. 2, the industrial robot 20 includes an attachment portion 21 which is attached to an installation portion 90 such as a floor or a ceiling, arms 22, 23, 24, 25, and 26, an articular portion 31 which connects the attachment portion 21 and the arm 22, an articular portion 32 which connects the arm 22 and the arm 23, an articular portion 33 which connects the arm 23 and the arm 24, an articular portion 34 which connects the arm 24 and the arm 25, an articular portion 35 which connects the arm 25 and the arm 26, and an articular portion 36 which connects the arm 26 and a hand (not shown).

Fig. 3 is a sectional view of the articular portion 32. Hereinafter, although the explanation is given of the articular portion 32, the configurations of the articular portions 31, and 33 to 36 are substantially same as the articular portion 32.

As shown in Fig. 3, the articular portion 32 includes a reducer 40 which connects the arm 22 and the arm 23, a motor 50 which is fixed to the arm 22 by bolts 51, and a lubricant deterioration sensor 60 which detects deterioration of lubricant 40a for reducing friction generated at the movable portions of the reducer 40, and a strain gauge 80 which is a load sensor for detecting a load applied to the reducer 40.

The reducer 40 includes a case 41 which includes an internal gear 41a and is fixed to the arm 22 by a bolt 41b, a supporting body 42 which includes three pillars 42a disposed with a constant interval around a center axis of the reducer 40 and is fixed to the arm 23 by a bolt 42b, a gear 43 which is fixed to an output shaft of the motor 50, three gears 44 which are disposed with a constant interval around the center axis of the reducer 40 and mesh with the gear 43, three crank shafts 45 which are disposed with a constant interval around the center axis of the reducer 40 and are fixed to the gears 44, and two external gears 46 which mesh with the internal gear 41a of the case 41.

The supporting body 42 is rotatably supported by the case 41 via bearings 41c. A seal member 41d for preventing leakage of the lubricant 40a is provided between the case 41 and the supporting body 42.

The crank shaft 45 is rotatably supported by the supporting body 42 via bearings 42c and rotatably supported by the external gears 46 via bearings 46a. The crank shaft 45 eccentrically rotates the external gear 46 with respect to the case 41 according to a rotational motion input from the gear 44.

The lubricant deterioration sensor 60 is fixed to the arm 23.

Two strain gauges 80 are attached to each of three pillars 42a of the supporting body 42 for a total of six. The strain gauge 80 detects a load applied to the reducer 40 as strain generated in pillar 42a. Here, a coating for protecting the strain gauge from the lubricant 40a is applied to the strain gauge 80. Moreover, in the reducer 40, a hole (not shown) for introducing a wire for transmitting the output of the strain gauge 80 to the outside is formed. In order to prevent the lubricant 40a from being leaked to the outside of the reducer 40, the hole is filled with a resin such as an epoxy based resin after the wire passes through the hole.

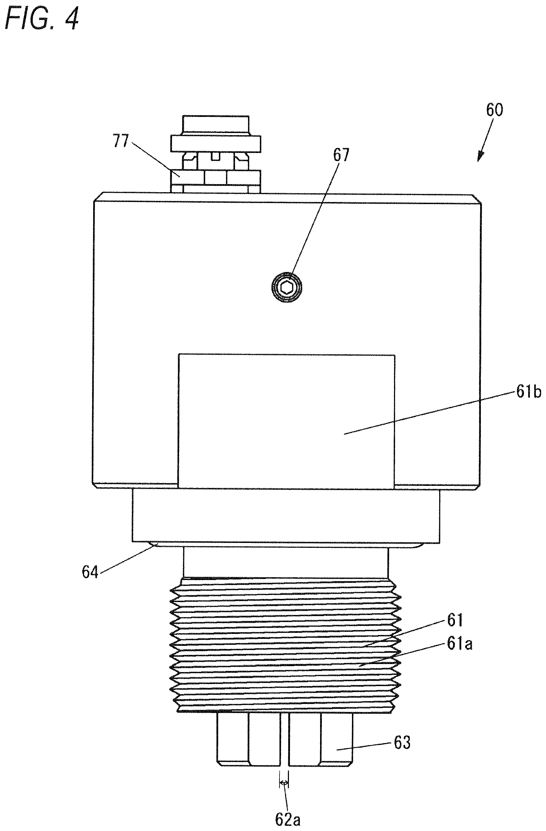

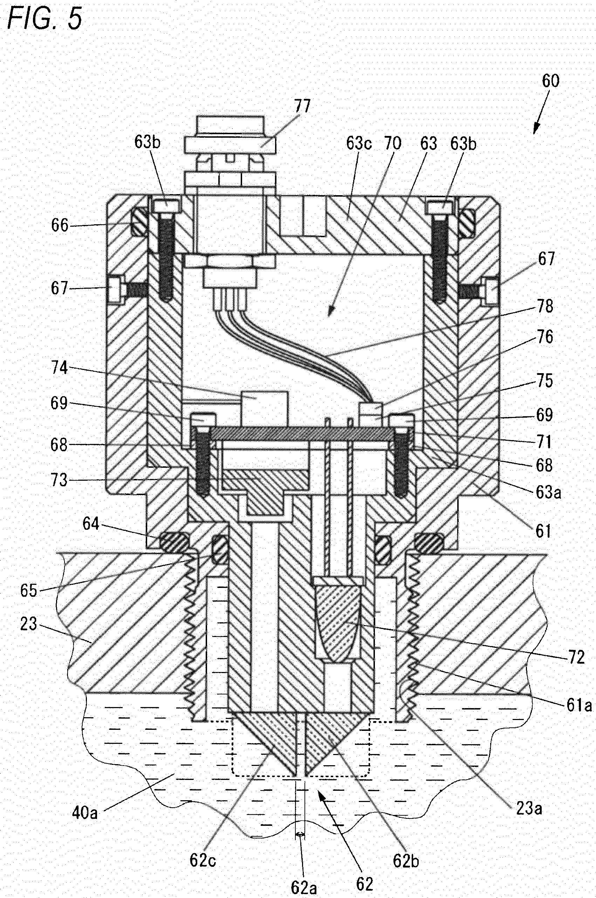

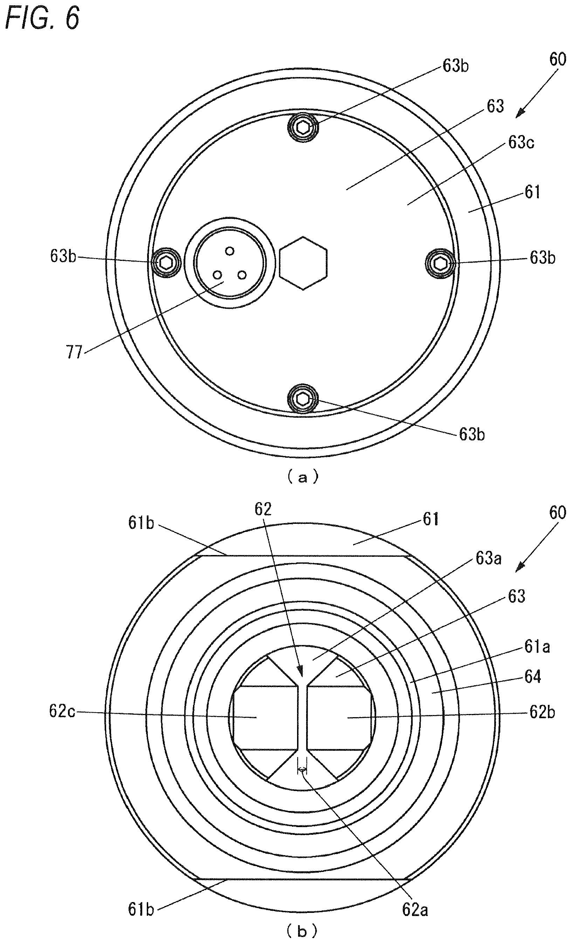

Fig. 4 is a front view of the lubricant deterioration sensor 60. Fig 5 is a front sectional view of the lubricant deterioration sensor 60 in a state being attached to the arm 23. Fig. 6(a) is a plan view of the lubricant deterioration sensor 60. Fig. 6(b) is a bottom view of the lubricant deterioration sensor 60.

As shown in Figs. 4 to 6(b), the lubricant deterioration sensor 60 includes a housing 61 made of aluminum alloy which supports the respective components of the lubricant deterioration sensor 60, a gap forming member 62 in which an oil gap 62a, which causes the lubricant 40a to enter therein, is formed, a supporting member 63 which supports the gap forming member 62, an O ring 64 which prevents the leakage of the lubricant 40a from a portion between the housing 61 and the arm 23, an O ring 65 which prevents the leakage of the lubricant 40a from a portion between the housing 61 and the supporting member 63, an O ring 66 which is disposed between the housing 61 and the supporting member 63, and an electronic component group 70.

The housing 61 includes a screw portion 61a which is formed to be fixed to the screw hole 23a of the arm 23, and a tool contact portion 61b which is formed to be grasped by a tool such as a spanner when the screw portion 61a is rotated with respect to the screw hole 23a of the arm 23. Moreover, in a state where the lubricant deterioration sensor 60 is removed, the screw hole 23a of the arm 23 may be used for supplying the lubricant 40a to the reducer 40 and for disposing the lubricant 40a from the reducer 40.

The gap forming member 62 is constituted of two right-angle prisms 62b and 62c made of glass, and the oil gap 62a which causes the lubricant 40a to enter therein is formed between the two right-angle prisms 62b and 62c. Each of the right-angle prisms 62b and 62c is fixed to the supporting member 63 by an adhesive.

The supporting member 63 is fixed to the housing 61 by bolts 67 with hexagonal holes. The supporting member 63 includes a holder 63a made of aluminum alloy, and a holder cap 63c made of aluminum alloy which is fixed to the holder 63a by bolts 63b with hexagonal holes.

The electronic component group 70 includes a circuit board 71 which is fixed to the supporting member 63 by the bolts 69 with hexagonal holes via spacers 68, a white LED 72 which is a light emitting element emitting white light and is mounted on the circuit board 71, an RGB sensor 73 which is a color light reception element detecting the color of received light and is mounted on the circuit board 71, a temperature sensor 74 which is a lubricant temperature sensor for detecting the temperature of the lubricant 40a via the holder 63a and is mounted on the circuit board 71, a connector 75 which is mounted on the circuit board 71 opposite to the white LED 72 and the RGB sensor 73 sides, a connector 76 which can be electrically connected to the connector 75, a waterproof connector 77 which is fixed to the holder cap 63c, and a plurality of lead wires 78 which electrically connect the connector 76 and the waterproof connector 77. A plurality of electronic components such as an electronic component processing signals of the white LED 72 and the RGB sensor 73 are mounted on the circuit board 71 in addition to the white LED 72, the RGB sensor 73, the temperature sensor 74, and the connector 75. A connector of the external device of the lubricant deterioration sensor 60 is connected to the waterproof connector 77, power is supplied from the external device via the connector of the external device, and the detected result of the lubricant deterioration sensor 60 is output to the external device via the connector of the external device as electric signals.

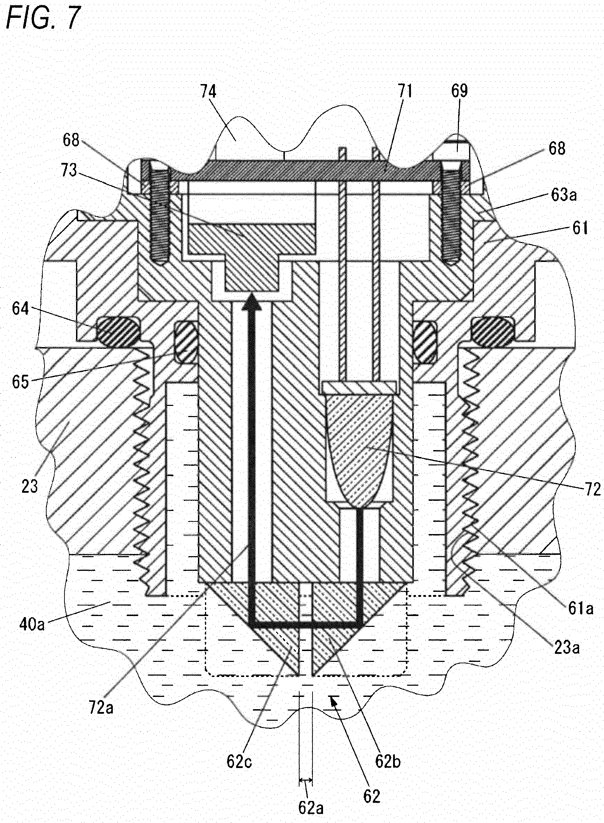

Fig. 7 is a view showing an optical path 72a from the white LED 72 to the RGB sensor 73.

As shown in Fig. 7, the oil gap 62a of the gap forming member 62 is disposed on the optical path 72a from the white LED 72 to the RGB sensor 73.

The holder 63a surrounds at least a portion of the optical path 72a from the white LED 72 to the RGB sensor 73. For example, the surface of the holder 63a is subjected to a treatment for preventing light reflection such as a black alumite treatment for matting.

The right-angle prisms 62b and 62c transmit the light emitted by the white LED 72. An incident surface and an emission surface of the light emitted by the white LED 72 in the right-angle prisms 62b and 62c are optically polished.

The optical path 72a is bent by 90° by the reflection surface of the right-angle prism 62b and also bent by 90° by the reflection surface of the right-angle prism 62c. That is, the optical path 72a is bent by 180° by the gap forming member 62. The reflection surfaces of the light emitted by the white LED 72 in the right-angle prisms 62b and 62c are optically polished, and an aluminum deposition film is formed on the reflection surfaces. Moreover, a SiO2 film is also formed on the aluminum deposition film to protect the aluminum deposition film which is low in a degree of hardness and an adhesive force.

A distance between the emission surface of the light emitted by the white LED 72 in the right-angle prism 62b and the incident surface of the light emitted by the white LED 72 in the right-angle prism 62c, that is, a length of the oil gap 62a, is 1 mm, for example. When the length of the oil gap 62a is too short, since the contaminant within the lubricant 40a does not easily and appropriately flow through the oil gap 62a, detection accuracy of the colors of the contaminant within the lubricant 40a degrades. On the other hand, when the length of the oil gap 62a is too long, since the light emitted from the white LED 72 is excessively absorbed by the contaminant within the lubricant 40a within the oil gap 62a and does not easily reach the RGB sensor 73, the detection accuracy of the colors of the contaminant within the lubricant 40a also degrades. Accordingly, preferably, the length of the oil gap 62a is appropriately set so that the detection accuracy of the colors of the contaminant within the lubricant 40a becomes high.

In the lubricant deterioration sensor 60, since the RGB sensor 73 detects colors with respect to the light having wavelengths not absorbed by the contaminant within the lubricant 40a in the oil gap 62a, among the white light emitted by the white LED 72, it is possible to immediately detect the colors of the contaminant within the lubricant 40a of the reducer 40. Moreover, the reducer damage state notification device 100 can immediately specify the kinds and amounts of the contaminant within the lubricant 40a of the reducer 40 based on the colors detected by the RGB sensor 73. That is, the lubricant deterioration sensor 60 detects the colors of the contaminant within the lubricant 40a, and thus, it is possible to detect a deterioration degree of the lubricant 40a.

In addition, the deterioration degree of the lubricant 40a can be determined by the color difference ΔE of the colors detected by the RGB sensor 73 with respect to black which is a predetermined color. The color difference ΔE of the color detected by the RGB sensor 73 with respect to black can be calculated by the following Expression 1 using each value of R, G, and B of the colors detected by the RGB sensor 73.

[Expression 1]

$$\Delta E = \sqrt{R^2 + G^2 + B^2}$$

Generally, in the industrial robot, accuracy in the locus of the arm or like largely depends on the performance of the reducer used in the articular portion. Accordingly, it is important to appropriately exchange the reducer for the industrial robot for a new one when the performance of the reducer degrades. However, in the case of exchanging the reducer for the industrial robot, it is necessary to stop the industrial robot provided with the reducer or a production line in which the industrial robot is installed. Accordingly, in order to grasp the exchange time of the reducer for the industrial robot, it is very important to appropriately predict a failure of the reducer for the industrial robot. Here, in each of the lubricant deterioration sensors of the industrial robot 20, the kinds and amounts of the contaminant within the lubricant 40a of the reducer 40 can be immediately specified by the reducer damage state notification device 100 based on the colors detected by the RGB sensor 73. Therefore, according to the machine system 10 with a reducer damage state notification function, it is possible to immediately predict the failure of the reducer for the industrial robot.

In addition, to the lubricant 40a, there may be added various kinds of additives such as a friction reducing agent like organic molybdenum (Mo) such as molybdenum dithiocarbamate (MoDTC) or molybdenum dithiophosphate (MoDTP) for reducing the friction of a friction surface, an extreme-pressure additive such as SP-based additive for improving extreme-pressure lubricity representing the performance for suppressing the seizure of the friction surface, and a dispersing agent such as calcium sulfonate for suppressing the generation and adhesion of sludge. For example, these additives adhere to, bind with or settle on the metal surface of the industrial robot 20 and the reducer in accordance with the deterioration of the lubricant 40a, and are separated from the lubricant 40a. Each of the lubricant deterioration sensors can specify, based on the detected colors, not only an amount of iron powder within the lubricant 40a but also a deterioration degree of the base oil and an increase of the contaminant such as sludge due to the reduction of various kinds of additives added to the lubricant 40a. Accordingly, the machine system 10 with a reducer damage state notification function can improve the prediction accuracy of a failure as compared with a technique where a failure of the reducer is predicted only based on a density of iron powder.

Here, an assembly method of the lubricant deterioration sensor 60 will be described.

First, the right-angle prisms 62b and 62c and the white LED 72 are fixed to the holder 63a by an adhesive.

Subsequently, the circuit board 71 on which the RGB sensor 73, the temperature sensor 74, and the connector 75 are mounted is fixed to the holder 63a by the bolts 69 with hexagonal holes via the spacer 68, and the white LED 72 is fixed to the circuit board 71 by soldering.

Subsequently, the temperature sensor 74 is connected to the holder 63a.

Subsequently, the connector 76, which is connected to the waterproof connector 77 fixed to the holder cap 63c via the lead wires 78, is connected to the connector 75 on the circuit board 71.

Subsequently, the holder cap 63c is fixed to the holder 63a by the bolts 63b with hexagonal holes.

Finally, the holder 63a is fixed to the housing 61, to which the O ring 64, the O ring 65, and the O ring 66 are attached, by bolts 67 with hexagonal holes.

In addition, a method of installing the lubricant deterioration sensor 60 to the arm 23 will be described.

First, the tool contact portion 61b of the housing 61 is grasped by a tool and the screw portion 61a of the housing 61 is screwed into the screw hole 23a of the arm 23, and thus, the lubricant deterioration sensor 60 is fixed to the arm 23.

In addition, the connector of the external device of the lubricant deterioration sensor 60 is connected to the waterproof connector 77.

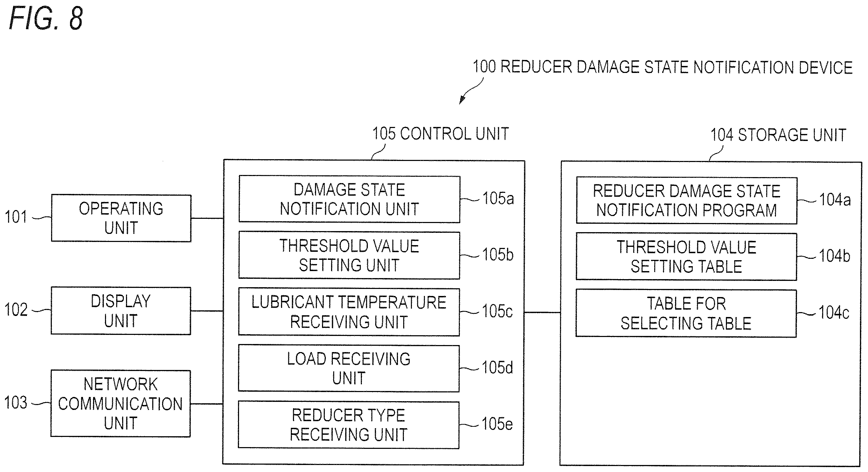

Fig. 8 is a block diagram of the reducer damage state notification device 100.

The reducer damage state notification device 100 shown in Fig. 8 is a personal computer (PC). The reducer damage state notification device 100 includes an operating unit 101 which is an input device such as a mouse or a keyboard to which various operations are input by a user, a display unit 102 which is a display device such as a liquid crystal display (LCD) on which various notification is displayed, a network communication unit 103 which is a network communication device performing communication via a network 11 (refer to Fig. 1), a storage unit 104 which is a storage device such as a hard disk drive (HDD) in which various data is stored, and a control unit 105 which controls the entire reducer damage state notification device 100.

The storage unit 104 stores a reducer damage state notification program 104a for sending the notification relating to the damage state of the reducer of the industrial robot 20.

The reducer damage state notification program 104a may be installed on the reducer damage state notification device 100 at a manufacturing step of the reducer damage state notification device 100, may be additionally installed on the reducer damage state notification device 100 from a recording medium such as a universal serial bus (USB) memory, a compact disc (CD), or a digital versatile disc (DVD), or may be additionally installed on the reducer damage state notification device 100 from a network 11.

The storage unit 104 stores a plurality of threshold value setting tables 104b which are tables for setting thresholds values to send the notification relating to the damage state of the reducer 40. Moreover, in the plurality of threshold value setting tables 104b stored in the storage unit 104, the types of the corresponding reducers are different from one another, and thus, ID is attached to each of the threshold value setting tables.

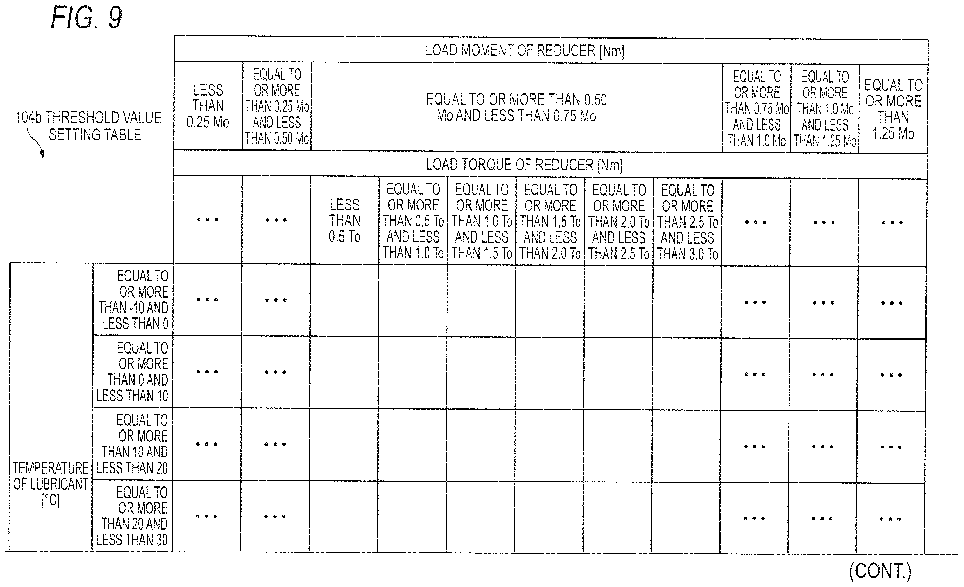

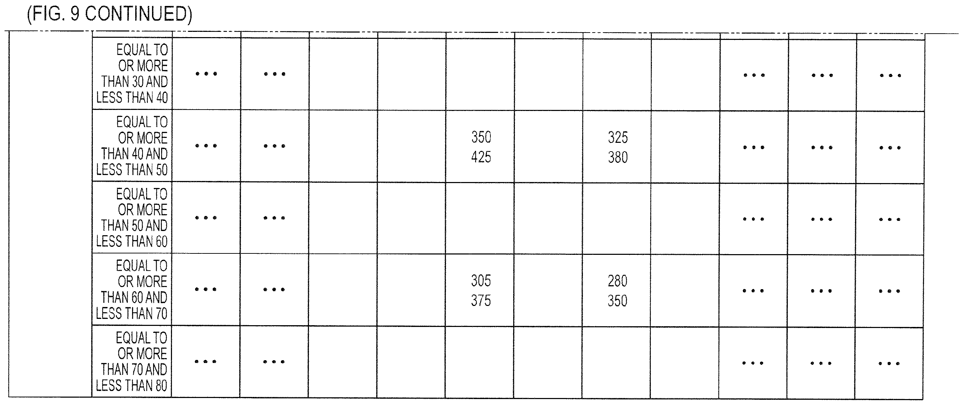

Fig. 9 is a table showing an example of the threshold value setting table 104b.

As shown in Fig. 9, in the threshold value setting tables 104b, thresholds values are shown, which correspond to the temperature of the lubricant 40a, a load (hereinafter, referred to as "load moment") which is applied to the reducer 40 in a rotation direction about an axis orthogonal to a rotation axis when the case 41 and the supporting body 42 are relatively rotated, and a load (hereinafter, referred to as "load torque") applied to the reducer 40 in the rotation direction about the rotation axis when the case 41 and the supporting body 42 are relatively rotated.

In the threshold value setting table 104b shown in Fig. 9, Mo indicates the rated moment of the reducer 40. In addition, To indicates the rated torque of the reducer 40.

Moreover, in the threshold value setting table 104b shown in Fig. 9, a plurality of blanks are present. However, actually, specific threshold values are written in the blanks. In addition, in the threshold value setting table 104b shown in Fig. 9, when the load moment of the reducer 40 is less than 0.25 Mo, when the load moment is equal to or more than 0.25 Mo and less than 0.5 Mo, when the load moment is equal to or more than 0.75 Mo and less than 1.0 Mo, when the load moment is equal to or more than 1.0 Mo and less than 1.25 Mo, and when the load moment is equal to or more than 1.25 Mo are shown to be omitted. However, actually, similar to when the load moment of the reducer 40 is equal to or more than 0.5 Mo and less than 0.75 Mo, specific threshold values are written in every range of the load torque of the reducer 40.

In the threshold value setting table 104b shown in Fig. 9, values are described in two lines in the columns of the threshold values. The upper value is a service and repair threshold value which is a threshold value for promoting service and repair to a user since the reducer 40 is very likely to be broken. The lower value is a warning threshold value which is a threshold value for warning that the reducer 40 is likely to be broken. For example, according to the threshold value setting table 104b shown in Fig. 9, when the temperature of the lubricant 40a is equal to or more than 40°C and less than 50°C, the load moment of the reducer 40 is equal to or more than 0.5 Mo and less than 0.75 Mo, and the load torque of the reducer 40 is equal to or more than 1.0 To and less than 1.5 To, the service and repair threshold value and the warning threshold value are 350 and 425, respectively.

Moreover, a case where the temperature of the lubricant 40a is less than -10°C and a case where the temperature of the lubricant 40a is equal to or more than 80°C are outside the specification range of the lubricant deterioration sensor according to the present embodiment, and thus, the above cases are not defined in the threshold value setting table 104b shown in Fig. 9. Moreover, a case where the load torque of the reducer 40 is equal to or more than 3.0 To is outside the specification range of the reducer according to the present embodiment, and thus, the case is not defined in the threshold value setting table 104b shown in Fig. 9.

As shown in Fig. 8, the storage unit 104 stores a table 104c for selecting a table which is a table for selecting an appropriate threshold value setting table among the plurality of threshold value setting tables 104b.

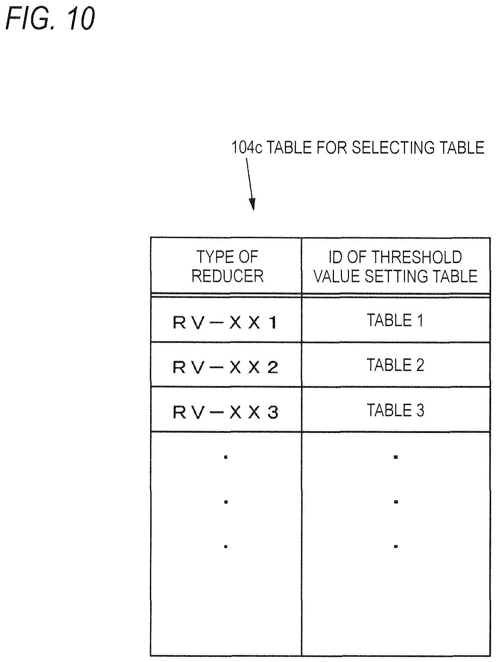

Fig. 10 is a table showing an example of the table 104c for selecting a table.

As shown in Fig. 10, in the table 104c for selecting a table, the ID of the threshold value setting table 104b corresponding to the type of the reducer is shown. For example, according to the table 104c for selecting a table 104c shown in Fig. 10, when the type of the reducer is "RV-XX2", the threshold value setting table is selected, in which the ID is "table 2" among the plurality of threshold value setting tables 104b.

For example, the control unit 105 shown in Fig. 8 includes a central processing unit (CPU), a read only memory (ROM) which stores programs and various data in advance, and a random access memory (RAM) which is used for an operation region of the CPU. The CPU executes the programs stored in the ROM or the storage unit 104.

The control unit 105 executes the reducer damage state notification programs 104a stored in the storage unit 104, and thus, functions as a damage state notification unit 105a which is damage state notification means for sending the notification relating to the damage state of the reducer 40, a threshold value setting unit 105b which is threshold value setting means for setting the threshold value for notification by the damage state notification unit 105a, a lubricant temperature receiving unit 105c which is lubricant temperature receiving means for receiving the temperature of the lubricant 40a, a load receiving unit 105d which is load receiving means for receiving the load applied to the reducer 40, and a reducer type receiving unit 105e which receives the type of the reducer 40.

Next, a calculation method of the load moment and the load torque by the load receiving unit 105d will be described.

Stresses, which are generated in portions to which two strain gauges 80 attached to one pillar 42a among three pillars 42a of the supporting body 42 of the reducer 40 adhere, are $\sigma 1$ and $\sigma 2$, respectively. The load torque calculated based on $\sigma 1$ and $\sigma 2$ is defined as $T_{A1}$. The load moment calculated based on the $\sigma 1$ and $\sigma 2$ is defined as $M_{A1}$, and a load coefficient in which the stress due to the load torque in the $\sigma 1$ can be calculated by multiplying $T_{A1}$ is defined as T1. The load coefficient in which the stress due to the load torque in the $\sigma 2$ can be calculated by multiplying $T_{A1}$ is defined as T2. An operation angle of the load moment applied to the supporting body 42 about the rotation axis of the supporting body 42 with respect to the case 41 is defined as $\varphi$. The load coefficient in which the stress due to the load moment in the $\sigma 1$ can be calculated by multiplying $M_{A1}$ is defined as $M1(\varphi)$ which is a function of $\varphi$. The load coefficient in which the stress due to the load moment in the $\sigma 2$ can be calculated by multiplying $M_{A1}$ is defined as $M2(\varphi)$ which is a function of $\varphi$. A relationship shown in Expression 2 is satisfied among $\sigma 1$, $\sigma 2$, $T_{A1}$, $M_{A1}$, T1, T2, M1(cp), and $M2(\varphi)$. In addition, the reason that $M1(\varphi)$ and $M2(\varphi)$ are functions of $\varphi$ is that the stress due to the load moment in $\sigma 1$ or the stress due to the load moment in $\sigma 2$ are changed according to the relative rotation between the case 41 and the supporting body 42.

[Expression 2]

$$\begin{Bmatrix} \sigma 1 \\ \sigma 2 \end{Bmatrix} = \begin{vmatrix} T1 & M1(\phi) \\ T2 & M2(\phi) \end{vmatrix} \begin{Bmatrix} T_{A1} \\ M_{A1} \end{Bmatrix}$$

Here, relationships shown in Expressions 3 and 4 are satisfied by Expression 2.

[Expression 3]

$$T_{A1} = \frac{\{\sigma1 \cdot M2(\phi) - \sigma2 \cdot M1(\phi)\}}{\{T1 \cdot M2(\phi) - T2 \cdot M1(\phi)\}}$$

[Expression 4]

$$M_{A1} = \frac{\{\sigma2 \cdot T1 - \sigma1 \cdot T2\}}{\{T1 \cdot M2(\phi) - T2 \cdot M1(\phi)\}}$$

Moreover, the stresses, which are generated in portions to which two strain gauges 80 attached to one pillar 42a other than the above-described pillar 42a among three pillars 42a of the supporting body 42 of the reducer 40 adhere, are $\sigma3$ and $\sigma4$, respectively. The load torque calculated based on $\sigma3$ and $\sigma4$ is defined as $T_{A2}$. The load moment calculated based on the $\sigma3$ and $\sigma4$ is defined as $M_{A2}$. The load coefficient in which the stress due to the load torque in the $\sigma3$ can be calculated by multiplying $T_{A2}$ is defined as T3. The load coefficient in which the stress due to the load torque in the $\sigma4$ can be calculated by multiplying $T_{A2}$ is defined as T4. The load coefficient in which the stress due to the load moment in the $\sigma1$ can be calculated by multiplying $M_{A1}$ is defined as M1($\phi$) which is a function of $\phi$. The load coefficient in which the stress due to the load moment in the $\sigma2$ can be calculated by multiplying $M_{A1}$ is defined as M2($\phi$) which is a function of $\phi$. A relationship shown in Expression 2 is satisfied among $\sigma1$, $\sigma2$, $T_{A1}$, $M_{A1}$, T1, T2, M1($\phi$), and M2($\phi$). In addition, the reason that M3($\varphi$) and M4($\varphi$) are functions of $\varphi$ is that the stress due to the load moment in $\sigma3$ or the stress due to the load moment in $\sigma4$ are changed according to the relative rotation between the case 41 and the supporting body 42.

[Expression 5]

$$\begin{Bmatrix} \sigma3 \\ \sigma4 \end{Bmatrix} = \begin{vmatrix} T3 & M3(\phi) \\ T4 & M4(\phi) \end{vmatrix} \begin{Bmatrix} T_{A2} \\ M_{A2} \end{Bmatrix}$$

Here, relationships shown in Expressions 6 and 7 are satisfied by Expression 5.

[Expression 6]

$$T_{A2} = \frac{\{\sigma3 \cdot M4(\phi) - \sigma4 \cdot M3(\phi)\}}{\{T3 \cdot M4(\phi) - T4 \cdot M3(\phi)\}}$$

[Expression 7]

$$M_{A2} = \frac{\{\sigma4 \cdot T3 - \sigma3 \cdot T4\}}{\{T3 \cdot M4(\phi) - T4 \cdot M3(\phi)\}}$$

Moreover, the stresses, which are generated in portions to which two strain gauges 80 attached to the remaining one pillar 42a other than the above-described two pillars 42a among three pillars 42a of the supporting body 42 of the reducer 40 adhere, are defined as $\sigma5$ and $\sigma6$, respectively. The load torque calculated based on $\sigma5$ and $\sigma6$ is defined as $T_{A3}$. The load moment calculated based on the $\sigma5$ and $\sigma6$ is defined as $M_{A3}$. The load coefficient in which the stress due to the load torque in the $\sigma5$ can be calculated by multiplying $T_{A3}$ is defined as T5. The load coefficient in which the stress due to the load torque in the $\sigma6$ can be calculated by multiplying $T_{A3}$ is defined as T6. The load coefficient in which the stress due to the load moment in the $\sigma5$ can be calculated by multiplying $M_{A3}$ is defined as M5($\varphi$) which is a function of $\varphi$. The load coefficient in which the stress due to the load moment in the $\sigma6$ can be calculated by multiplying

$M_{A3}$ is defined as M6($\varphi$) which is a function of $\varphi$. A relationship shown in Expression 8 is satisfied among $\sigma5$, $\sigma6$, $T_{A3}$, $M_{A3}$, T5, T6, M5($\varphi$), and M6($\varphi$). In addition, the reason that M5 ($\varphi$) and M6($\varphi$) are functions of $\varphi$ is that the stress due to the load moment in $\sigma5$ or the stress due to the load moment in $\sigma6$ are changed according to the relative rotation between the case 41 and the supporting body 42.

[Expression 8]

$$\left\{\begin{matrix} \sigma5 \\ \sigma6 \end{matrix}\right\} = \left|\begin{matrix} T5 & M5(\phi) \\ T6 & M6(\phi) \end{matrix}\right| \left\{\begin{matrix} T_{A3} \\ M_{A3} \end{matrix}\right\}$$

Here, relationships shown in Expressions 9 and 10 are satisfied by Expression 8.

[Expression 9]

$$T_{A3} = \frac{\{\sigma5 \cdot M6(\phi) - \sigma6 \cdot M5(\phi)\}}{\{T5 \cdot M6(\phi) - T6 \cdot M5(\phi)\}}$$

[Expression 10]

$$M_{A3} = \frac{\{\sigma6 \cdot T5 - \sigma5 \cdot T6\}}{\{T5 \cdot M6(\phi) - T6 \cdot M5(\phi)\}}$$

Moreover, theoretically, since $T_{A1}$, $T_{A2}$, and $T_{A3}$ are values which are the same as one another, $\varphi$ when $T_{A1}$, $T_{A2}$, and $T_{A3}$ are the value which is closest to one another is closest to the actual operation angle of the load moment. Similarly, theoretically, since $M_{A1}$, $M_{A2}$, and $M_{A3}$ are values which are the same as one another, $\varphi$ when the $M_{A1}$, $M_{A2}$, and $M_{A3}$ are the value which is closest to one another is closest to the actual operation angle of the load moment. That is, $\varphi$ when the function $\delta(\varphi)$ shown in Expression 11 becomes the minimum value is closest to the actual operation angle of the load moment.

[Expression 11]

$$\delta(\phi) = \frac{(T_{A1} - T_{A2})^2}{(T_{A1} + T_{A2})^2} + \frac{(T_{A2} - T_{A3})^2}{(T_{A2} + T_{A3})^2} + \frac{(T_{A3} - T_{A1})^2}{(T_{A3} + T_{A1})^2}$$
$$+ \frac{(M_{A1} - M_{A2})^2}{(M_{A1} + M_{A2})^2} + \frac{(M_{A2} - M_{A3})^2}{(M_{A2} + M_{A3})^2} + \frac{(M_{A3} - M_{A1})^2}{(M_{A3} + M_{A1})^2}$$

Accordingly, the load receiving unit 105d calculates $\varphi$ when the function $\delta(\varphi)$ obtains the minimum value based on Expressions 3, 4, 6, 7, 9, 10, and 11, and thus, $\varphi$ closest to the actual operation angle of the load moment is calculated. Here, the load receiving unit 105d can calculate $\sigma1$ to $\sigma6$ based on the output of the strain gauge 80. Moreover, T1 to T6 and M1($\varphi$) to M6($\varphi$) are measured in advance by a load test.

Next, the load receiving unit 105d calculates $T_{A1}$, $T_{A2}$, $T_{A3}$, $M_{A1}$, $M_{A2}$, and $M_{A3}$ from Expressions 3, 4, 6, 7, 9, and 10 based on the calculated $\varphi$.

Finally, the load receiving unit 105d calculates T which is the load torque applied to the reducer 40 and M which is the load moment applied to the reducer 40 based on the calculated $T_{A1}$, $T_{A2}$, $T_{A3}$, $M_{A1}$, $M_{A2}$, and $M_{A3}$, from Expressions 12 and 13.

[Expression 12]

$$T = (T_{A1} + T_{A2} + T_{A3})/3$$

[Expression 13]

$$M = (M_{A1} + M_{A2} + M_{A3})/3$$

Next, an example of a preparation method of the threshold value setting table 104b will be described.

In a device for the experimentation prepared so as to have the same configuration as that of the articular portion 32, under a condition that the maximum output rotation speed was 15 rpm, a reciprocal rotation movement of rotating the supporting body 42 by 45° in a reverse direction with respect to the case 41 after rotating the supporting body 42 by 45° in a forward direction with respect to the case 41 was continued. In this experiment, new oil with a low deterioration degree was used as the lubricant 40a. Moreover, the chorological change of the color difference ΔE of the color detected by the RGB sensor 73 of the lubricant deterioration sensor 60 with respect to black was investigated.

Fig. 11(a) is a graph showing an example of the experimentation result of the chorological change of the color difference ΔE when the temperature of the lubricant 40a is 40°C, the load moment of the reducer 40 is 0.5 Mo, and the load torque of the reducer 40 is 1.0 To. Fig. 11(b) is a graph showing an example of the experimentation result of the chorological change of the color difference ΔE when the temperature of the lubricant 40a is 60°C, the load moment of the reducer 40 is 0.5 Mo, and the load torque of the reducer 40 is 1.0 To. Fig. 11(c) is a graph showing an example of the experimentation result of the chorological change of the color difference ΔE when the temperature of the lubricant 40a is 60°C, the load moment of the reducer 40 is 0.5 Mo, and the load torque of the reducer 40 is 2.0 To.

In Figs. 11(a) to 11(c), a rated conversion time was obtained in a manner such that a time during which the device for the experimentation was actually driven was converted into a time of a case where the output rotation speed is 15 rpm and the load torque is the rated torque of the reducer 40, based on the output rotation speed and the load torque of the reducer 40 in a case where the device for the experimentation was actually driven. Moreover, the life time of the reducer 40 is defined as 6,000 hours in a case where the reducer 40 is continuously driven under a condition that the output rotation speed is 15 rpm and the load torque is the rated torque of the reducer 40.

As shown in Figs. 11(a) and 11(b), when the temperatures of the lubricant 40a are different from each other although the load moment and the load torque of the reducer 40 are the same as each other, the color differences ΔE (hereinafter, referred to as a "color difference during rated life time") at the time when the rated conversion time reaches 6000 hours which is the life time are different from each other. Moreover, as shown in Figs. 11(b) and 11(c), when the load torques of the reducer 40 are different from each other although the temperature of the lubricant 40a and the load moment of the reducer 40 are the same as each other, the color differences during the rated life time are different from each other. According to the similar experimentation, when any of the temperature of the lubricant 40a, the load moment of the reducer 40, and the load torque of the reducer 40 is different, it can be confirmed that the color differences during the rated life time are different from each other. Accordingly, preferably, the threshold value for sending the notification relating to the damage state of the reducer 40 is determined in association with the temperature of the lubricant 40a, the load moment of the reducer 40, and the load torque of the reducer 40.

In addition, when a plurality of experimentations are performed, although the temperature of the lubricant 40a, the load moment of the reducer 40, and the load torque of the reducer 40 are the same as one another in each experimentation, deviation is generated in the color difference during the rated life time. Accordingly, among the color differences during the related life time in the plurality of experimentations, the minimum value is defined as the service and repair threshold value, and the maximum value is defined as the warning threshold value.

For example, in the threshold value setting table 104b shown in Fig. 9, 350 which is the service and repair threshold value when the temperature of the lubricant is equal to or more than 40°C and less than 50°C, the load moment of the reducer is equal to or more than 0.5 Mo and less than 0.75 Mo, and the load torque of the reducer is equal to or more than 1.0 To and less than 1.5 To is the minimum value among the color differences during the rated life time in the plurality of experimentations when the plurality of experimentations are performed under a condition that the temperature of the lubricant is 40°C, the load moment of the reducer is 0.5 Mo, and the load torque of the reducer is 1.0 To. Similarly, in the threshold value setting table 104b shown in Fig. 9, 425 which is the warning threshold value when the temperature of the lubricant is equal to or more than 40°C and less than 50°C, the load moment of the reducer is equal to or more than 0.5 Mo and less than 0.75 Mo, and the load torque of the reducer is equal to or more than 1.0 To and less than 1.5 To is the maximum value among the color differences during the rated life time in the plurality of experimentations when the plurality of experimentations are performed under a condition that the temperature of the lubricant is 40°C, the load moment of the reducer is 0.5 Mo, and the load torque of the reducer is 1.0 To.

The threshold value setting table 104b is prepared as described above. Moreover, the experimentation is performed under a condition that the types of the reducers are different from each other, and thus, a plurality of threshold value setting tables 104b are prepared.

Next, the operation of the machine system 10 with a reducer damage state notification function will be described.

First, the operation of the industrial robot 20 will be described.

Moreover, hereinafter, although the explanation is made to the articular portion 32, the articular portions 31, 33 to 36 are similar to the articular portion 32.

When the output shaft of the motor 50 of the articular portion 32 rotates, the rotation speed of the motor 50 is reduced by the reducer 40, and thus, the arm 23 fixed to the supporting body 42 of the reducer 40 is moved with respect to the arm 22 fixed to the case 41 of the reducer 40.

The lubricant deterioration sensor 60 of the articular portion 32 emits white light from the white LED 72 by the power supplied from the external device via the waterproof connector 77. Moreover, the lubricant deterioration sensor 60 outputs the light amount of each color in RGB of the light received by the RGB sensor 73 to the external device via the waterproof connector 77 as an electric signal. In addition, the lubricant deterioration sensor 60 outputs the temperature detected by the temperature sensor 74 to the external device via the waterproof connector 77 as an electric signal.

In addition, the strain detected by the strain gauge 80 is also output to the external device of the reducer 40.

Next, the operation of the reducer damage state notification device 100 will be described.

Moreover, hereinafter, although the explanation is made to the lubricant deterioration sensor 60 of the articular portion 32, the lubricant deterioration sensors of the articular portions 31, 33 to 36 are also similar to the lubricant deterioration sensor of the articular portion 32.

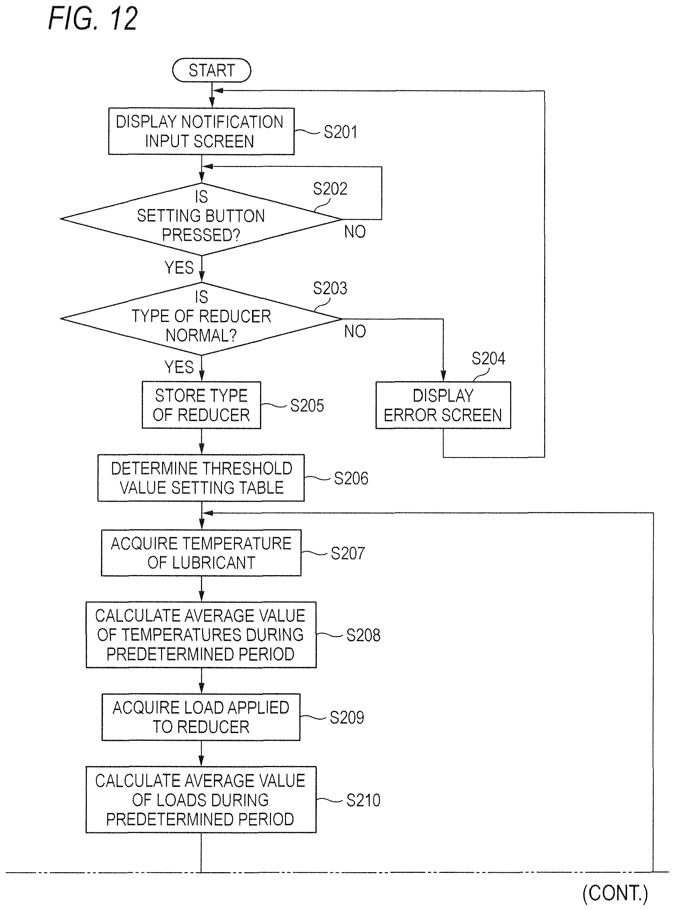

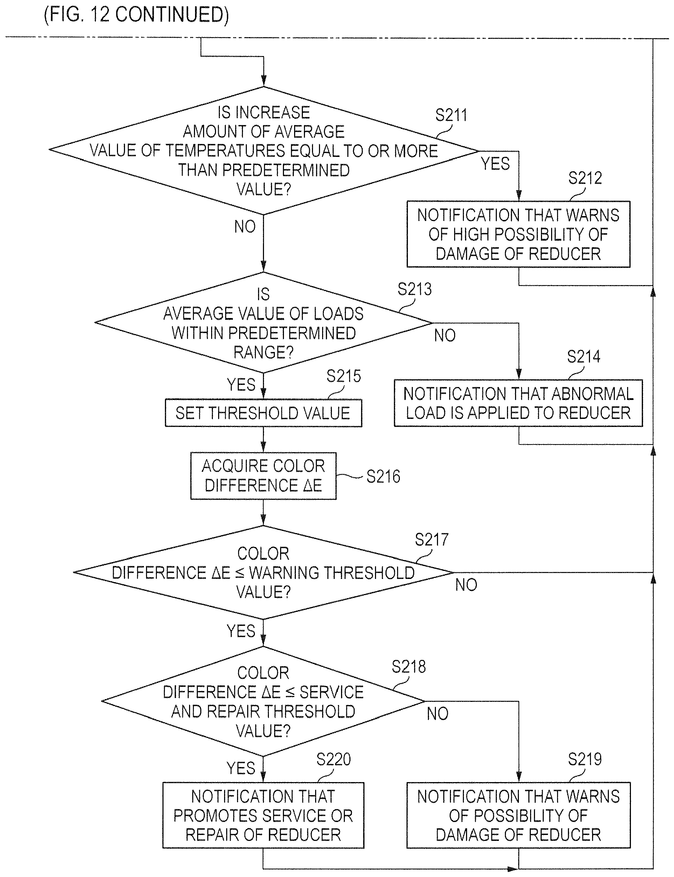

Fig. 12 is a flowchart of the operation of the reducer damage state notification device 100.

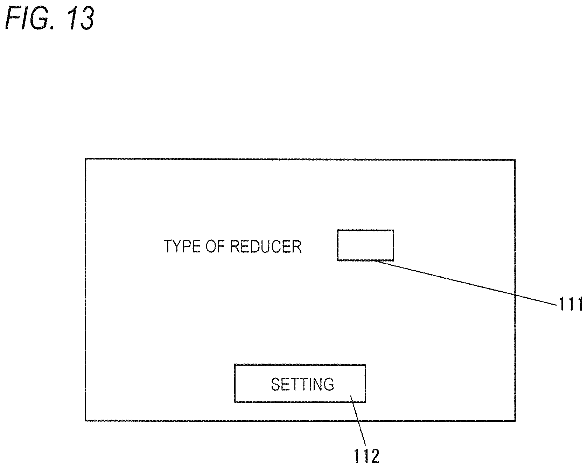

As shown in Fig. 12, the control unit 105 of the reducer damage state notification device 100 displays the notification input screen shown in Fig. 13 which is the screen for inputting the type of the reducer 40 on the display unit 102 (S201).

Fig. 13 is a diagram showing an example of the notification input screen displayed on the display unit 102.

The notification input screen shown in Fig. 13 includes a text box 111 for inputting the type of the reducer 40, and a setting button 112 which is a button for setting the notification input to the text box 111. The user inputs appropriate notification to the text box 111 via the operating unit 101 and can press the setting button 112.

As shown in Fig. 12, the control unit 105 determines whether or not the setting button 112 is pressed until it is determined that the setting button 112 is pressed (S202).

The reducer type receiving unit 105e of the control unit 105 determines whether or not the type of the reducer 40 input to the text box 111 is normal if it is determined at S202 that the setting button 112 is pressed (S203). Here, the reducer type receiving unit 105e determines that the type of the reducer 40 input to the text box 111 is normal only when the type of the reducer 40 included in the table 104c for selecting a table on the storage unit 104 is input to the text box 111.

In Step S203, when it is determined that the type of the reducer 40 input to the text box 111 is not normal, the reducer type receiving unit 105e displays the error screen on the display unit 102 (S204), and this step returns to the processing of S201 again.

On the other hand, in Step S203, when it is determined that the type of the reducer 40 input to the text box 111 is normal, the reducer type receiving unit 105e stores the type of the reducer 40 input to the text box 111 (S205). That is, the reducer type receiving unit 105e receives the type of the reducer 40.

Subsequently, the control unit 105 acquires the ID of the corresponding threshold value setting table 104b in the table 104c for selecting a table on the storage unit 104 with respect to the type of the reducer 40 stored in the S205, and determines the threshold value setting table 104b to which the acquired ID is attached as the threshold value setting table used in the following processing (S206).

Subsequently, the lubricant temperature receiving unit 105c of the control unit 105 acquires the detected result of the temperature sensor 74 of the lubricant deterioration sensor 60 via the network communication unit 103 (S207). That is, the lubricant temperature receiving unit 105c receives the temperature of the lubricant 40a.

Subsequently, for example, the control unit 105 calculates an average value of the temperatures of the lubricant 40a which are acquired in a plurality of times of S207 during a predetermined period such as 10 minutes (S208). That is, the control unit 105 calculates the average value of the temperature of the lubricant 40a based on not only the temperature of the lubricant 40a acquired in the immediately preceding S207 but also the temperature of the lubricant 40a acquired in one or more times of the last S207 which are performed before the immediately preceding S207.

Subsequently, the load receiving unit 105d of the control unit 105 acquires the detected result of the strain gauge 80 via the network communication unit 103, calculates the load moment and the load torque of the reducer 40 as described above based on the detected result of the acquired strain gauge 80, and thus, acquires the load moment and the load torque of the reducer 40 (S209). That is, the load receiving unit 105d receives the load moment and the load torque of the reducer 40.

Subsequently, for example, the control unit 105 calculates an average value of each of the load moment and the load torque of the reducer 40 which are acquired in a plurality of times of S209 during a predetermined period such as 10 minutes (S210). That is, the control unit 105 calculates the average value of each of the load moment and the load torque of the reducer 40 based on not only the load moment and the load torque of the reducer 40 acquired in the immediately preceding S209 but also the load moment and the load torque of the reducer 40 acquired in one or more times of the last S209 which are performed before the immediately preceding S209.

Next, the damage state notification unit 105a of the control unit 105 determines whether or not the increase amount of the average value in the temperatures of the lubricant 40a calculated in S208 is equal to or more than a predetermined value such as 20°C or more, for example (S211).

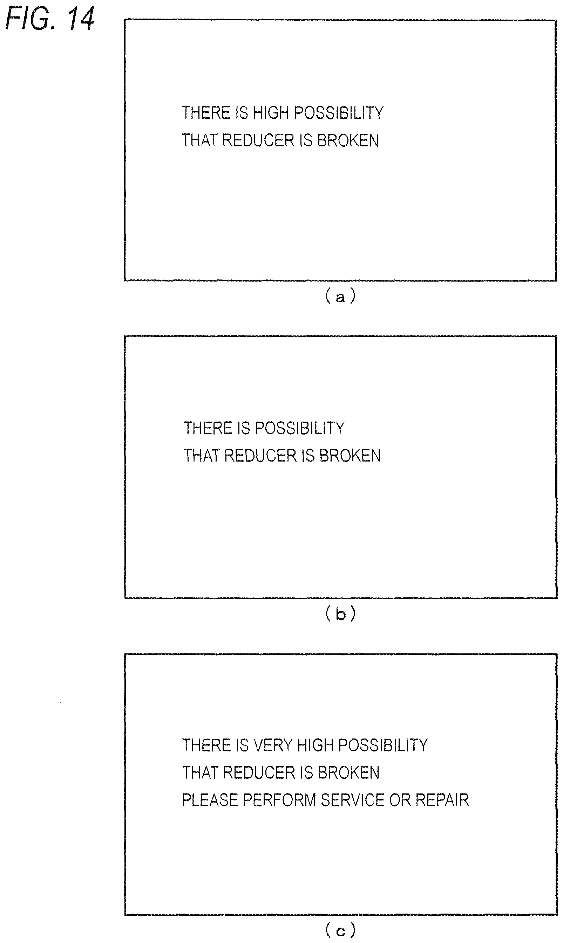

In Step S211, when it is determined that the increase amount of the average value in the temperatures of the lubricant 40a calculated in S208 is equal to or more than the predetermined value, as shown in Fig. 14(a), the damage state notification unit 105a displays the notification that warns of a high possibility of the damage of the reducer 40 on the display unit 102 as the notification of the damage state of the reducer 40 (S212), and the step returns to the processing of S207 again.

On the other hand, in Step S211, when it is determined that the increase amount of the average value in the temperatures of the lubricant 40a calculated in S208 is less than the predetermined value, the control unit 105 determines whether or not the average value of the load moment and the average value of the load torque of the reducer 40 calculated in S210 are within a predetermined range (S213).

In S213, when it is determined that the average value of the load moment or the average value of the load torque of the reducer 40 calculated in S210 is not within a predetermined range, the control unit 105 notifies the display unit 102 of an abnormal load being applied to the reducer 40(S214), and the step returns to the processing of S207 again.

On the other hand, in S213, when it is determined that the average value of the load moment and the average value of the load torque of the reducer 40 calculated in S210 are within the predetermined range, the threshold value setting unit 105b of the control unit 105 sets the corresponding warning threshold value and service and repair threshold in the threshold value setting table 104b determined in S206 with respect to the average value of the temperatures of the lubricant 40a calculated in S208, the average value of the load moment of the reducer 40 calculated in S210, and the average value of the load torque of the reducer 40 calculated in S210, as the threshold values used in the following processing (S215).

Subsequently, the damage state notification unit 105a acquires the detected result of the RGB sensor 73 of the lubricant deterioration sensor 60, that is, the color of the lubricant 40a via the network communication unit 103, and calculates to acquire the color difference ΔE of the acquired color with respect to black by the above-described Expression 1 (S216).

Subsequently, the damage state notification unit 105a determines whether or not the color difference ΔE acquired in S216 is less than or equal to the warning threshold value set in S215 (S217).

In S217, when the damage state notification unit 105a determines that the color difference ΔE acquired in S216 is more than the warning threshold value set in S215, the step returns to the processing of S207 again.

On the other hand, in S217, when it is determined that the color difference ΔE acquired in S216 is less than or equal to the warning threshold value set in S215, the damage state notification unit 105a determines whether or not the color difference ΔE acquired in S216 is less than or equal to the service and repair threshold value set in S215 (S218).

In S218, when it is determined that the color difference ΔE acquired in S216 is more than the service and repair threshold value set in S215, as shown in Fig. 14(b), the damage state notification unit 105a displays the notification that warns of a possibility of the damage of the reducer 40 on the display unit 102, as the notification of the damage state of the reducer 40 (S219). That is, when the color difference ΔE between the color detected by the RGB sensor 73 and black reaches the warning threshold value, the damage state notification unit 105a sends the notification relating to the damage state of the reducer 40 corresponding to the warning threshold value. After the control unit 105 performs the processing of S219, the step returns to the processing of S207 again.

On the other hand, in S218, when it is determined that the color difference ΔE acquired in S216 is less than or equal to the service and repair threshold value set in S215, as shown in Fig. 14(c), the damage state notification unit 105a displays the notification that promotes the service or repair of the reducer 40 to the user on the display unit 102 as the notification of the damage state of the reducer 40 (S220). That is, when the color difference ΔE between the color detected by the RGB sensor 73 and black reaches the service and repair threshold value, the damage state notification unit 105a sends the notification relating to the damage state of the reducer 40 corresponding to the service and repair threshold value. After the control unit 105 performs the processing of S220, the step returns to the processing of S207 again.

As described above, the reducer damage state notification device 100 sets the warning threshold value and the service and repair threshold value according to the temperature (S207 and S208) of the lubricant 40a and the load applied to the reducer 40, that is, the load moment and the load torque (S209 and S210) of the reducer 40 (S215), and thus, it is possible to accurately send the notification relating to the damage state of the reducer 40 even when the temperatures of the lubricant 40a and the loads applied to the reducer 40 are different from each other as the use condition of the reducer 40. In addition, compared to a configuration in which the reducer damage state notification device 100 sets the warning threshold value and the service and repair threshold value according to all of the temperature of the lubricant 40a, the load moment of the reducer 40, and the load torque of the reducer 40, even when the reducer damage state notification device 100 does not perform the setting of the warning threshold value and the service and repair threshold value according to one or two of the temperature of the lubricant 40a, the load moment of the reducer 40, and the load torque of the reducer 40, the accuracy is decreased. However, it is possible to accurately send the notification relating to the damage state of the reducer 40.

The reducer damage state notification device 100 sets the warning threshold value and the service and repair threshold value according to the actual temperature (S207) of the lubricant 40a detected by the temperature sensor 74 (S215), and thus, it is possible to accurately send the notification relating to the damage state of the reducer 40 according to the actual temperature of the lubricant 40a. In addition, the reducer damage state notification device 100 may set the warning threshold value and the service and repair threshold value according to the temperature of the lubricant 40a other than the temperature of the lubricant 40a detected by the temperature sensor 74. For example, the reducer damage state notification device 100 may set the warning threshold value and the service and repair threshold value according to the temperature of the lubricant 40a input from the user via the operating unit 101.

The reducer damage state notification device 100 sets the warning threshold value and the service and repair threshold value according to the load detected by the strain gauge 80, that is, the load (S209) actually applied to the reducer 40 (S215), and thus, it is possible to accurately send the notification relating to the damage state of the reducer 40 according to the load actually applied to the reducer 40. In addition, the method of detecting the load actually applied to the reducer 40 may adopt a method other than the strain gauge 80.

The reducer damage state notification device 100 sets the warning threshold value and the service and repair threshold value according to the average value (S207 and S208) of the actual temperature of the lubricant 40a during a predetermined period (S215), and thus, it is possible to prevent the notification relating to the damage state of the reducer 40 from being erroneously sent when the temperature of the lubricant 40a is temporarily and suddenly changed. Moreover, the reducer damage state notification device 100 may adopt only the actual temperature of the lubricant 40a acquired in the immediately preceding S207, and not the average value of the actual temperatures of the lubricant 40a during the predetermined period, as the temperature of the lubricant 40a used in S215.

The reducer damage state notification device 100 sets the warning threshold value and the service and repair threshold value according to the average value (S209 and S210) of the loads actually applied to the reducer 40 during a predetermined period (S215), and thus, it is possible to prevent the notification relating to the damage state of the reducer 40 from being erroneously sent when the load applied to the reducer 40 is temporarily and suddenly changed. Moreover, the reducer damage state notification device 100 may adopt only the load acquired in the immediately preceding S209, and not the average value of the loads actually applied to the reducer 40 during the predetermined period, as the load used in S215.

Immediately after the lubricant 40a is exchanged with a new one or the like, the deterioration of the lubricant 40a may not be generated immediately even when the reducer 40 actually approaches the damage or the reducer 40 is broken already. If the deterioration of the lubricant 40a is not generated, the color difference $\Delta E$ between the color detected by the RGB sensor 73 and black does not reach the warning threshold value and the service and repair threshold value. However, when the reducer 40 actually approaches the damage or the reducer 40 is broken already, the reducer 40 is intensively heated, and thus, the temperature of the lubricant 40a is suddenly increased. The reducer damage state notification device 100 sends the notification relating to the damage state of the reducer 40 (S212) when the increase amount of the average value (S207 and S208) of the actual temperatures of the lubricant 40a during the predetermined period is equal to or more than the predetermined value (YES in S211), and thus, although the color difference $\Delta E$ between the color detected by the RGB sensor 73 and black does not reach the warning threshold value and the service and repair threshold value even when the reducer 40 actually approaches the damage or the reducer 40 is broken already, it is possible to appropriately send the notification relating to the damage state of the reducer 40.

When it is not possible to send the notification relating to the damage state of the reducer 40 and the load applied to the reducer 40 is not within the predetermined range (NO in S213), the reducer damage state notification device 100 can notify the notification that an abnormal load is applied to the reducer 40 (S214).

In addition, after the reducer damage state notification device 100 performs the processing of S210 without performing the processing of S211 to S214, the device 100 may immediately perform the processing of S215.

The temperature sensor 74 detects the temperature of the lubricant 40a through the holder 63a. However, the temperature sensor may directly detect the temperature of the lubricant 40a without going through other members such as the holder 63a.

Compared to a configuration in which the temperature sensor 74 is separately provided on the lubricant deterioration sensor 60, the temperature sensor 74 is incorporated in the lubricant deterioration sensor 60, and thus, it is possible easily to structure the machine system 10 with a reducer damage state notification function. In addition, in the machine system 10 with a reducer damage state notification function, the temperature sensor 74 may be separately provided on the lubricant deterioration sensor 60.

The reducer damage state notification device 100 may not perform the setting of the warning threshold value and the service and repair threshold value according to the type of the reducer 40.

The reducer damage state notification device 100 includes two levels of threshold values corresponding to the possibility of the damage of the reducer 40, that is, the warning threshold value and the service and repair threshold value, as the threshold value for sending the notification relating to the damage state of the reducer 40, and thus, it is possible to send the notification relating to the damage state of the reducer 40 in two levels as the possibility of the damage of the reducer 40 (S219 and S220). For example, compared to a configuration in which notification relating to a very high possibility of the damage of the reducer 40 is abruptly sent, since the notification of the damage state of the reducer 40 is sent in a plurality of steps as the possibility of the damage of the reducer 40, the user can determine the necessity of the exchange of the reducer 40 with sufficient time. Moreover, the reducer damage state notification device 100 may have only one threshold value as the threshold value for sending the notification relating to the damage state of the reducer 40, and may have three more levels or of threshold values corresponding to the possibility of the damage of the reducer 40 as the threshold value for sending the notification relating to the damage state of the reducer 40.

The reducer damage state notification device 100 performs the notification by means of the display in the present embodiment. However, in addition to the display, or instead of the display, for example, the reducer damage state notification device may perform the notification by means of methods other than the display such as a sound output.

For example, each lubricant deterioration sensor such as the lubricant deterioration sensor 60 may use a battery such as a cell as the supplying means of power, and, for example, may also use wireless communication as the output means of the detected result to the external device.

The reducer damage state notification device of the present invention is the PC for executing the reducer damage state notification program in the present embodiment. However, the reducer damage state notification device may be realized by other configurations. For example, the reducer damage state notification device of the present invention may be realized by a control panel which controls the operation of the industrial robot 20, and may be realized by a computer which manages the operation of the plurality of industrial robot 20.

The machine of the present invention is the industrial robot in the present embodiment. However, the machine of the present invention may be the machine other than the industrial robot.

Priority is claimed on Japanese Patent Application No. 2012-113127, filed on May 17, 2012, the content of which is incorporated herein by reference.

Industrial Applicability

According to the present invention, it is possible to provide a reducer damage state notification device capable of accurately sending the notification relating to the damage state of the reducer even when the use conditions of the reducer are different from each other.

Reference Signs List

10: machine system with reducer damage state notification function
20: industrial robot (machine)
40: reducer
40a: lubricant
41: case
41a: internal gear
42: supporting body
45: crank shaft
46: external gear
60: lubricant deterioration sensor
62: gap forming member
62a: oil gap
72: white LED (light emitting element)
72a: optical path
73: RGB sensor (color light reception element)
74: temperature sensor (lubricant temperature sensor)
80: strain gauge (load sensor)
100: reducer damage state notification device (computer)
104a: reducer damage state notification program
105a: damage state notification unit (damage state notification means)
105b: threshold value setting unit (threshold value setting means)
105c: lubricant temperature receiving unit (lubricant temperature receiving means)
105d: load receiving unit (load receiving means)

**Claims**

1. A reducer damage state notification device (100) for sending notification relating to a damage state of a reducer (40) of a machine (20) which includes the reducer (40) and a lubricant deterioration sensor (60) for detecting deterioration of lubricant (40a) of the reducer (40),
wherein the lubricant deterioration sensor (60) includes a light emitting element (72) which emits light, a color light reception element (73) which detects color of received light, and a gap forming member (62) in which an oil gap (62a), which the lubricant (40a) enters and is disposed on an optical path (72a) from the light emitting element (72) to the color light reception element (73), is formed, and by which the light is transmitted, **characterized in that**, the reducer damage state notification device (100) comprising:

   a damage state notification means (105a) for sending notification relating to the damage state of the reducer (40);
   a threshold value setting means (105b) for setting a threshold value for notification by the damage state notification means (105a); and
   a load receiving means (105d) for receiving a load which is applied to the reducer (40), wherein the damage state notification means (105a) sends notification relating to the damage state of the reducer (40) corresponding to the threshold value when a color difference between the color detected by the color light reception element (73) and a predetermined color reaches the threshold value set by the threshold value setting means (105b), and the threshold value setting means (105b) sets the threshold value according to the load which is received by the load receiving means.

2. The reducer damage state notification device (100) according to Claim 1,
wherein the reducer (40) includes:

   a case (41) which includes an internal gear (41a);
   a supporting body (42) which is rotatably supported by the case (41);
   an external gear (46) which meshes with the internal gear (41a); and
   a crank shaft (45) which is rotatably supported by the supporting body (42) and the external gear (46) and is configured to eccentrically rotate the external gear (46) with respect to the case (41) according to a rotational motion input from the outside,
   wherein the load receiving means (105d) receives at least one of a load which is applied to the reducer (40) in a rotation direction about a rotation axis when the case (41) and the supporting body (42) are relatively rotated, and a load which is applied to the reducer (40) in a rotation direction about an axis orthogonal to the rotation axis.

3. The reducer damage state notification device (100) according to Claim 1, wherein
the machine (20) includes a load sensor (80) which is configured to detect the load applied to the reducer (40), and
the load receiving means (105d) receives the load which is detected by the load sensor (80).

4. The reducer damage state notification device (100) according to Claim 3, wherein
the threshold value setting means (105b) sets the threshold value according to an average value of predetermined periods of the load which is received by the load receiving means (105d).

5. The reducer damage state notification device (100) according to any one of Claims 1 to 4, further comprising
a lubricant temperature receiving means (105c) for receiving a temperature of the lubricant (40a),
wherein the threshold value setting means (105b) sets the threshold value according to the temperature of the lubricant (40a) which is received by the lubricant temperature receiving means (105c).

6. The reducer damage state notification device (100) according to Claim 5, wherein
the machine (20) includes a lubricant temperature sensor (74) which is configured to detect the temperature of the lubricant (40a), and
the lubricant temperature receiving means (105c) receives the temperature of the lubricant (40a) which is detected by the lubricant temperature sensor (74).

7. The reducer damage state notification device (100) according to Claim 6, wherein
the threshold value setting means (105b) sets the threshold value according to an average value of the temperature of the lubricant (40a) which is received by the lubricant temperature receiving means (105c) during predetermined periods.

8. The reducer damage state notification device (100) according to any one of Claims 1 to 7, wherein the threshold value includes a plurality of levels of threshold values corresponding to a possibility of the damage of the reducer (40).

9. A machine system with a reducer damage state notification function (10), comprising:

   the reducer damage state notification device (100) according to any one of Claims 1 to 8; and
   the machine (20) which is configured so that the notification relating to the damage state of the reducer (40) is sent by the reducer damage state notification device (100).

10. A machine system with a reducer damage state notification function (10), comprising:

   the reducer damage state notification device (100) according to Claim 6 or 7; and
   the machine (20) in which the notification relating to the damage state of the reducer (40) is sent by the reducer damage state notification device (100),
   wherein the lubricant temperature sensor (74) is incorporated in the lubricant deterioration sensor (60).

11. A medium storing a reducer damage state notification program (104a) which causes a computer to function as the reducer damage state notification device (100) according to any one of Claims 1 to 8.

**Patentansprüche**

1. Untersetzungsschadenszustands-Benachrichtigungsvorrichtung (100), um eine Benachrichtigung bezüglich eines Schadenszustands einer Untersetzung (40) einer Maschine (20) zu senden, die die Untersetzung (40) und einen Schmiermittelverschlechterungssensor (60) zum Erkennen einer Verschlechterung des Schmiermittels (40a) der Untersetzung (40) umfasst,
   wobei der Schmiermittelverschlechterungssensor (60) umfasst: ein lichtemittierendes Element (72), das Licht emittiert, ein Farblichtempfangselement (73), das die Farbe des empfangenen Lichts erfasst, und ein Spaltbildungselement (62), in dem ein Ölspalt (62a) ausgebildet ist, in den das Schmiermittel (40a) eintritt, und das auf einem optischen Weg (72a) von dem lichtemittierenden Element (72) zu dem Farblichtempfangselement (73) angeordnet ist, und durch das das Licht übertragen wird,
   **dadurch gekennzeichnet, dass**
   die Untersetzungsschadenszustands-Benachrichtigungsvorrichtung (100) umfasst:

   eine Schadenszustands-Benachrichtigungseinrichtung (105a) zum Senden einer Benachrichtigung bezüglich des Schadenszustands der Untersetzung (40);
   eine Schwellenwerteinstelleinrichtung (105b) zum Einstellen eines Schwellenwertes zur Benachrichtigung durch die Schadenszustands-Benachrichtigungseinrichtung (105a); und
   eine Lastaufnahmeeinrichtung (105d) zum Aufnehmen einer Last, die auf die Untersetzung (40) wirkt, wobei die Schadenszustands-Benachrichtigungseinrichtung (105a) eine Benachrichtigung bezüglich des Schadenszustands der Untersetzung (40) sendet, die dem Schwellenwert entspricht, wenn eine Farbdifferenz zwischen der durch das Farblichtempfangselement (73) erfassten Farbe und einer vorbestimmten Farbe den durch die Schwellenwerteinstelleinrichtung (105b) eingestellten Schwellenwert erreicht, und die Schwellenwerteinstelleinrichtung (105b) den Schwellenwert gemäß der Last einstellt, die von der Lastaufnahmeeinrichtung aufgenommen wird.

2. Untersetzungsschadenszustands-Benachrichtigungsvorrichtung (100) nach Anspruch 1, bei der die Untersetzung (40) umfasst:

   ein Gehäuse (41), das ein Innenzahnrad (41a) umfasst;
   einen Haltekörper (42), der drehbar von dem Gehäuse (41) gehalten ist;
   ein Außenzahnrad (46), das in das Innenzahnrad (41a) eingreift; und
   eine Kurbelwelle (45), die drehbar durch den Haltekörper (42) und das Außenzahnrad (46) gelagert und dazu eingerichtet ist, das Außenzahnrad (46) in Bezug auf das Gehäuse (41) gemäß einer Drehbewegungseingabe von außen exzentrisch zu drehen,
   wobei die Lastaufnahmeeinrichtung (105d) eine Last, die auf die Untersetzung (40) in einer Drehrichtung um eine Drehachse ausgeübt wird, wenn das Gehäuse (41) und der Haltekörper (42) relativ gedreht werden, und/oder eine Last aufnimmt, die auf das Untersetzungsgetriebe (40) in einer Drehrichtung um eine zur Drehachse orthogonale Achse wirkt.

3. Untersetzungsschadenszustands-Benachrichtigungsvorrichtung (100) nach Anspruch 1, bei der die Maschine (20) einen Lastsensor (80) umfasst, der dazu eingerichtet ist, die an dem Untersetzer (40) angelegte Last zu erfassen, und die Lastaufnahmeeinrichtung (105d) die Last aufnimmt, die vom Lastsensor (80) erfasst wird.

4. Untersetzungsschadenszustands-Benachrichtigungsvorrichtung (100) nach Anspruch 3, bei der die Schwellenwerteinstelleinrichtung (105b) den Schwellenwert gemäß einem Durchschnittswert von vorbestimmten Zeitperioden der Last einstellt, die von der Lastaufnahmeeinrichtung (105d) aufgenommen wird.

5. Untersetzungsschadenszustands-Benachrichtigungsvorrichtung (100) nach einem der Ansprüche 1 bis 4, ferner umfassend
eine Schmiermitteltemperatur-Aufnahmeeinrichtung (105c) zum Aufnehmen einer Temperatur des Schmiermittels (40a),
wobei die Schwellenwert-Einstelleinrichtung (105b) den Schwellenwert gemäß der Temperatur des Schmiermittels (40a) einstellt, die von der Schmiermitteltemperatur-Empfangseinrichtung (105c) empfangen wird.

6. Untersetzungsschadenszustands-Benachrichtigungsvorrichtung (100) nach Anspruch 5, bei der die Maschine (20) einen Schmiermitteltemperatursensor (74) umfasst, der dazu eingerichtet ist, die Temperatur des Schmiermittels (40a) zu erfassen, und die Schmiermitteltemperatur-Empfangseinrichtung (105c) die Temperatur des Schmiermittels (40a) empfängt, die von dem Schmiermitteltemperatursensor (74) erfasstwird.

7. Untersetzungsschadenszustands-Benachrichtigungsvorrichtung (100) nach Anspruch 6, bei der die Schwellenwert-Einstelleinrichtung (105b) den Schwellenwert gemäß einem Durchschnittswert der Temperatur des Schmiermittels (40a) einstellt, der von der Schmiermitteltemperatur-Empfangseinrichtung (105c) während vorbestimmter Perioden empfangen wird.

8. Untersetzungsschadenszustands-Benachrichtigungsvorrichtung (100) nach einem der Ansprüche 1 bis 7, bei der der Schwellenwert eine Vielzahl von Graden von Schwellenwerten umfasst, die der Möglichkeit der Beschädigung der Untersetzung (40) entsprechen.

9. Maschinensystem mit einer Untersetzungsschadenszustands-Benachrichtigungsfunktion (10), umfassend:

die Untersetzungsschadenszustands-Benachrichtigungsvorrichtung (100) nach einem der Ansprüche 1 bis 8; und
die Maschine (20), die so konfiguriert ist, dass die Benachrichtigung bezüglich des Schadenszustands der Untersetzung (40) von der Untersetzungsschadenszustands-Benachrichtigungsvorrichtung (100) gesendet wird.

10. Maschinensystem mit einer Untersetzungsschadenszustands-Benachrichtigungsfunktion (10), umfassend:

eine Untersetzungsschadenszustands-Benachrichtigungsvorrichtung (100) gemäß Anspruch 6 oder 7; und
die Maschine (20), bei der die Benachrichtigung bezüglich des Schadenszustands der Untersetzung (40) von der Untersetzungsschadenszustands-Benachrichtigungsvorrichtung (100) gesendet wird,
wobei der Schmiermitteltemperatursensor (74) in den Schmiermittelverschlechterungssensor (60) eingebaut ist.

11. Medium, das ein Untersetzungsschadenszustands-Benachrichtigungsprogramm (104a) speichert, das bewirkt, dass ein Computer als die Untersetzungsschadenszustands-Benachrichtigungsvorrichtung (100) nach einem der Ansprüche 1 bis 8 arbeitet.

**Revendications**

1. Dispositif de notification de l'état de dégradation d'un réducteur (100) permettant d'envoyer une notification concernant l'état de dégradation d'un réducteur (40) d'une machine (20) qui comprend le réducteur (40) et un capteur de dégradation de lubrifiant (60) permettant de détecter la dégradation de lubrifiant (40a) du réducteur (40), dans lequel le capteur de dégradation de lubrifiant (60) comprend un élément électroluminescent (72) qui émet de la lumière, un élément de réception de lumière colorée (73) qui détecte la couleur de la lumière reçue et un élément de formation d'intervalle (62) dans lequel est constitué un intervalle d'huile (62a), dans laquelle le lubrifiant (40a) entre et est disposé sur un chemin optique (72a) allant de l'élément électroluminescent (72) à l'élément de réception de lumière de couleur (73), et par lequel la lumière est transmise, **caractérisé en ce que** le dispositif de notification de l'état de dégradation d'un réducteur (100) comprenant:

un moyen de notification de l'état de dégradation (105a) pour envoyer une notification relative à l'état de dégradation du réducteur (40);

un moyen d'établissement de valeur de seuil (105b) pour établir une valeur seuil pour une notification par le moyen de notification de l'état de dégradation (105a); et

un moyen de réception de charge (105d) permettant de recevoir une charge qui est appliquée au réducteur (40), le moyen de notification de l'état de dégradation (105a) envoyant une notification relative à l'état de dégradation du réducteur (40) correspondant à la valeur de seuil lorsqu'une différence de couleur entre la couleur détectée par l'élément de réception de lumière de couleur (73) et une couleur prédéterminée atteint la valeur de seuil définie par le moyen d' établissement de valeur de seuil (105b), et

le moyen d'établissement de valeur de seuil (105b) établit la valeur de seuil en fonction de la charge reçue par le moyen de réception de charge.

2. Dispositif de notification de l'état de dégradation d'un réducteur (100) selon la revendication 1, dans lequel le réducteur (40) comprend:

un carter (41) qui comprend un engrenage interne (41a);

un corps de support (42) qui est pris en charge de manière rotative par le carter (41);

un engrenage externe (46) qui se met en prise avec l'engrenage interne (41a); et

un vilebrequin (45) qui est pris en charge de manière rotative par le corps de support (42) et l'engrenage externe (46) et est configuré pour faire tourner de manière excentrique l'engrenage externe (46) par rapport au carter (41) en fonction d'un mouvement de rotation saisi de l'extérieur,

dans lequel le moyen de réception de charge (105d) reçoit au moins une charge parmi une charge qui est appliquée au réducteur (40) dans un sens de rotation autour d'un axe de rotation lorsque le carter (41) et le corps de support (42) sont soumis à une rotation relative, et une charge qui est appliquée au réducteur (40) dans un sens de rotation autour d'un axe orthogonal à l'axe de rotation.

3. Dispositif de notification de l'état de dégradation d'un réducteur (100) selon la revendication 1, dans lequel la machine (20) comprend un capteur de charge (80) qui est configuré pour détecter la charge appliquée au réducteur (40), et

le moyen de réception de charge (105d) reçoit la charge qui est détectée par le capteur de charge (80).

4. Dispositif de notification de l'état de dégradation d'un réducteur (100) selon la revendication 3, dans lequel le moyen d'établissement de valeur seuil (105b) établit la valeur seuil en fonction d'une valeur moyenne de périodes prédéterminées de la charge qui est reçue par le moyen de réception de charge (105d).

5. Dispositif de commande de déplacement (100) selon l'une quelconque des revendications 1 à 4, comprenant en outre un moyen de réception de température de lubrifiant (105c) pour recevoir une température du lubrifiant (40a), dans lequel le moyen d'établissement de valeur seuil (105b) établit la valeur seuil en fonction de la température du lubrifiant (40a) qui est reçue par le moyen de réception de température de lubrifiant (105c).

6. Dispositif de notification de l'état de dégradation d'un réducteur (100) selon la revendication 5, dans lequel la machine (20) comprend un capteur de température de lubrifiant (74) qui est configuré pour détecter la température du lubrifiant (40a), et

le moyen de réception de température de lubrifiant (105c) reçoit la température du lubrifiant (40a) qui est détectée par le capteur de température de lubrifiant (74).

7. Dispositif de notification de l'état de dégradation d'un réducteur (100) selon la revendication 6, dans lequel le moyen d'établissement de valeur seuil (105b) établit la valeur seuil en fonction d'une valeur moyenne de la température du lubrifiant (40a) qui est reçue par le moyen de réception de température de lubrifiant (105c) pendant des périodes prédéterminées.

**8.** Dispositif de commande de déplacement (100) selon l'une quelconque des revendications 1 à 7, dans lequel la valeur de seuil comprend une pluralité de niveaux de valeurs seuil correspondant à une possibilité de dégradation du réducteur (40).

**9.** Système de machine avec une fonction de notification de l'état de dégradation d'un réducteur (10), comprenant:

le dispositif de notification de l'état de dégradation d'un réducteur (100) selon l'une quelconque des revendications 1 à 8;
et
la machine (20) qui est configurée pour que la notification relative à l'état d'endommagement du réducteur (40) soit envoyée par le dispositif de notification de l'état de dégradation d'un réducteur (100).

**10.** Système de machine avec une fonction de notification de l'état de dégradation d'un réducteur (10), comprenant:

le dispositif de notification de l'état de dégradation d'un réducteur (100) selon les revendications 6 ou 7; et
la machine (20) dans laquelle la notification relative à l'état de dégradation du réducteur (40) est envoyée par le dispositif de notification de l'état de dégradation d'un réducteur (100),
dans lequel le capteur de température de lubrifiant (74) est intégré au capteur de dégradation du lubrifiant (60).

**11.** Support stockant un programme de notification de l'état de dégradation d'un réducteur (104a) qui conduit un ordinateur à fonctionner comme dispositif de notification de l'état de dégradation d'un réducteur (100) selon l'une quelconque des revendications 1 à 8.

EP 2 851 675 B1

*FIG. 1*

10 MACHINE SYSTEM WITH REDUCER DAMAGE
STATE NOTIFICATION FUNCTION

20

11

100

| INDUSTRIAL ROBOT |
| --- |

NETWORK

| REDUCER DAMAGE STATE
NOTIFICATION DEVICE |
| --- |

FIG. 2

FIG. 3

FIG. 4

FIG. 5

## FIG. 6

( a )

( b )

FIG. 7

*FIG. 8*

100 REDUCER DAMAGE STATE NOTIFICATION DEVICE

105 CONTROL UNIT

104 STORAGE UNIT

| | |
|---|---|
| 101 — OPERATING UNIT | |

DAMAGE STATE NOTIFICATION UNIT — 105a

THRESHOLD VALUE SETTING UNIT — 105b

102 — DISPLAY UNIT

LUBRICANT TEMPERATURE RECEIVING UNIT — 105c

LOAD RECEIVING UNIT — 105d

103 — NETWORK COMMUNICATION UNIT

REDUCER TYPE RECEIVING UNIT — 105e

REDUCER DAMAGE STATE NOTIFICATION PROGRAM — 104a

THRESHOLD VALUE SETTING TABLE — 104b

TABLE FOR SELECTING TABLE — 104c

## FIG. 9

104b THRESHOLD VALUE SETTING TABLE

| | | LOAD MOMENT OF REDUCER [Nm] | | | | | | | | | |
|---|---|---|---|---|---|---|---|---|---|---|---|
| | | LESS THAN 0.25 Mo | EQUAL TO OR MORE THAN 0.25 Mo AND LESS THAN 0.50 Mo | EQUAL TO OR MORE THAN 0.50 Mo AND LESS THAN 0.75 Mo | | | | | | EQUAL TO OR MORE THAN 0.75 Mo AND LESS THAN 1.0 Mo | EQUAL TO OR MORE THAN 1.0 Mo AND LESS THAN 1.25 Mo | EQUAL TO OR MORE THAN 1.25 Mo |
| | | LOAD TORQUE OF REDUCER [Nm] | | | | | | | | | | |
| | | ... | ... | LESS THAN 0.5 To | EQUAL TO OR MORE THAN 0.5 To AND LESS THAN 1.0 To | EQUAL TO OR MORE THAN 1.0 To AND LESS THAN 1.5 To | EQUAL TO OR MORE THAN 1.5 To AND LESS THAN 2.0 To | EQUAL TO OR MORE THAN 2.0 To AND LESS THAN 2.5 To | EQUAL TO OR MORE THAN 2.5 To AND LESS THAN 3.0 To | ... | ... | ... |
| TEMPERATURE OF LUBRICANT [°C] | EQUAL TO OR MORE THAN -10 AND LESS THAN 0 | ... | ... | | | | | | | ... | ... | ... |
| | EQUAL TO OR MORE THAN 0 AND LESS THAN 10 | ... | ... | | | | | | | ... | ... | ... |
| | EQUAL TO OR MORE THAN 10 AND LESS THAN 20 | ... | ... | | | | | | | ... | ... | ... |
| | EQUAL TO OR MORE THAN 20 AND LESS THAN 30 | ... | ... | | | | | | | ... | ... | ... |

(CONT.)

EP 2 851 675 B1

(FIG. 9 CONTINUED)

| | EQUAL TO OR MORE THAN 30 AND LESS THAN 40 | ... | ... | | | | | | | ... | ... | ... |
|---|---|---|---|---|---|---|---|---|---|---|---|---|
| | EQUAL TO OR MORE THAN 40 AND LESS THAN 50 | ... | ... | | | 350 425 | | 325 380 | | ... | ... | ... |
| | EQUAL TO OR MORE THAN 50 AND LESS THAN 60 | ... | ... | | | | | | | ... | ... | ... |
| | EQUAL TO OR MORE THAN 60 AND LESS THAN 70 | ... | ... | | | 305 375 | | 280 350 | | ... | ... | ... |
| | EQUAL TO OR MORE THAN 70 AND LESS THAN 80 | ... | ... | | | | | | | ... | ... | ... |

## FIG. 10

104c TABLE FOR SELECTING TABLE

| TYPE OF REDUCER | ID OF THRESHOLD VALUE SETTING TABLE |
|:---:|:---:|
| R V − X X 1 | TABLE 1 |
| R V − X X 2 | TABLE 2 |
| R V − X X 3 | TABLE 3 |
| . . . | . . . |

# FIG. 11

(a)

(b)

(c)

## FIG. 12

START

DISPLAY NOTIFICATION INPUT SCREEN — S201

IS SETTING BUTTON PRESSED? — S202 — NO

YES

IS TYPE OF REDUCER NORMAL? — S203 — NO

YES

STORE TYPE OF REDUCER — S205

DISPLAY ERROR SCREEN — S204

DETERMINE THRESHOLD VALUE SETTING TABLE — S206

ACQUIRE TEMPERATURE OF LUBRICANT — S207

CALCULATE AVERAGE VALUE OF TEMPERATURES DURING PREDETERMINED PERIOD — S208

ACQUIRE LOAD APPLIED TO REDUCER — S209

CALCULATE AVERAGE VALUE OF LOADS DURING PREDETERMINED PERIOD — S210

(CONT.)

(FIG. 12 CONTINUED)

IS INCREASE AMOUNT OF AVERAGE VALUE OF TEMPERATURES EQUAL TO OR MORE THAN PREDETERMINED VALUE?  S211

YES

NO

NOTIFICATION THAT WARNS OF HIGH POSSIBILITY OF DAMAGE OF REDUCER  S212

IS AVERAGE VALUE OF LOADS WITHIN PREDETERMINED RANGE?  S213

NO

YES

SET THRESHOLD VALUE  S215

NOTIFICATION THAT ABNORMAL LOAD IS APPLIED TO REDUCER  S214

ACQUIRE COLOR DIFFERENCE ΔE  S216

COLOR DIFFERENCE ΔE ≤ WARNING THRESHOLD VALUE?  S217

NO

YES

COLOR DIFFERENCE ΔE ≤ SERVICE AND REPAIR THRESHOLD VALUE?  S218

NO

YES

NOTIFICATION THAT PROMOTES SERVICE OR REPAIR OF REDUCER  S220

NOTIFICATION THAT WARNS OF POSSIBILITY OF DAMAGE OF REDUCER  S219

*FIG. 13*

TYPE OF REDUCER

SETTING

111

112

*FIG. 14*

THERE IS HIGH POSSIBILITY
THAT REDUCER IS BROKEN

( a )

THERE IS POSSIBILITY
THAT REDUCER IS BROKEN

( b )

THERE IS VERY HIGH POSSIBILITY
THAT REDUCER IS BROKEN
PLEASE PERFORM SERVICE OR REPAIR

( c )

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2447704 A1
- JP 7146233 A
- JP 10104160 A
- JP 2012113127 A

**Non-patent literature cited in the description**

- **TOMOHIKO YAMAGUCHI.** METHOD OF DISCRIMINATING HUE OF CONTAMINANT WITHIN LUBRICANT. Faculty of Engineering, March 2003, vol. 51, 81-88
- **TOMOMI HONDA.** DETERIORATION DIAGNOSIS OF LUBRICANT · INSPECTION TECHNOLOGY. *Journal of Japan Society for Precision Engineering,* 2009, vol. 75 (3), 359-362